(19) Europäisches Patentamt / European Patent Office / Office européen des brevets

(11) **EP 3 869 196 B1**

(12) **EUROPEAN PATENT SPECIFICATION**

(45) Date of publication and mention
of the grant of the patent:
**15.11.2023 Bulletin 2023/46**

(51) International Patent Classification (IPC):
*G01N 33/543* (2006.01)

(52) Cooperative Patent Classification (CPC):
**G01N 33/54313**

(21) Application number: **21165034.6**

(22) Date of filing: **07.11.2014**

(54) **QUANTITATIVE CONTROLS AND CALIBRATORS FOR CELLULAR ANALYTES**

QUANTITATIVE STEUERUNGEN UND KALIBRATOREN FÜR ZELLULÄRE ANALYTEN

TÉMOINS QUANTITATIFS ET ÉTALONNEURS POUR ANALYTES CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2013 US 201361901394 P**

(43) Date of publication of application:
**25.08.2021 Bulletin 2021/34**

(62) Document number(s) of the earlier application(s) in
accordance with Art. 76 EPC:
**19159269.0 / 3 514 543**
**14859458.3 / 3 065 704**

(73) Proprietor: **Boston Cell Standards Inc.**
**Sharon, MA 02067 (US)**

(72) Inventors:
• **SOMPURAM, Seshi R**
**Arlington, MA 02474 (US)**
• **BOGEN, Steven A**
**Sharon, MA 02067 (US)**

(74) Representative: **D Young & Co LLP**
**120 Holborn**
**London EC1N 2DY (GB)**

(56) References cited:
WO-A1-97/24613    WO-A1-2004/077057
WO-A2-00/62064

• SOMPURAM SESHI R ET AL: "A novel quality control slide for quantitative immunohistochemistry testing", JOURNAL OF HISTOCHEMISTRY AND CYTOCHEMISTRY, HISTOCHEMICAL SOCIETY, NEW YORK, NY, US, vol. 50, no. 11, 1 January 2002 (2002-01-01), pages 1425-1433, XP002569974, ISSN: 0022-1554
• SOMPURAM SESHI R ET AL: "Abstract", CLINICAL CHEMISTRY, vol. 48, no. 3, 1 March 2002 (2002-03-01), pages 410-420, XP055816083, US ISSN: 0009-9147, DOI: 10.1093/clinchem/48.3.410 Retrieved from the Internet: URL:https://watermark.silverchair.com/clin chem0410.pdf?token=AQECAHi208BE49Ooan9 kkhW _Ercy7Dm3ZL_9Cf3qfKAc485ysgAAAsQwggLA Bgkqh kiG9w0BBwagggKxMIICrQIBADCCAqYGCSqGS Ib3DQE HATAeBglghkgBZQMEAS4wEQQMhX8yQhKz58 F8mLv5A gEQglICd2N47siQX0PZV6X9oXGs2vuBuHMbYY 6jY1P n1PftmvSrT-NP5mZJCIIC3FjFq5vVPs8ZQPoxL_ xYc BXuf6DG0se>

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 869 196 B1

**Description**

**CLAIM OF PRIORITY**

This application claims priority to U.S. Provisional Application 61/901,394, filed November 7, 2013.

**TECHNICAL FIELD**

The present invention relates to quality control and calibration of advanced staining procedures in the field of histopathology. Common examples of these advanced staining procedures include immunohistochemistry and in situ hybridization.

**BACKGROUND**

Measurement of cellular analytes

Immunohistochemical (IHC) stains are an integral part of the diagnostic process in surgical pathology. IHC stains are typically used with conventional histopathological stains, as adjunctive assays. Traditional (non-IHC) microscopic analysis of biopsy samples demonstrates overall cellular and tissue architecture. With the commonly used hematoxylin and eosin stain, for example, nuclei are colored purple (with hematoxylin) and the cytoplasm red/pink (with eosin). Often, the type of information available from such stains is insufficient for patient management. IHC stains have the ability to extend the level of analysis on a biopsy sample, beyond cellular size and shape, to a protein level. With IHC staining, tissue samples can be probed for the presence and quantity of specific proteins. The presence of a particular protein in an IHC test causes a colored reaction product, which can be evaluated by microscopic examination of the tissue section. For example, the presence of certain proteins can be indicative of the cellular lineage of a tumor, helping the pathologist reach a proper diagnosis. Alternatively, the presence of microbiologic agents, such as viruses or bacteria can be detected. IHC stains are also often used to determine an appropriate course of treatment. Examples of such stains include those for human epidermal growth factor receptor type 2 (HER-2), estrogen receptor (ER), and progesterone receptor (PR) analysis for breast carcinoma. An additional type of advanced staining method in histopathology is in situ hybridization, which measures specific nucleic acids instead of proteins.

Quality control for immunohistochemistry

To assure that clinical test results are accurate, controls are run with all in vitro diagnostic tests. Quality control in the clinical laboratory is mandated by the Clinical Laboratory Improvement Act of 1988 (CLIA '88). This act outlines the regulatory requirements that a clinical laboratory must meet in order to obtain accreditation. Many tests in the clinical laboratory, for sections other than histopathology (such as the hematology and chemistry sections), include controls and calibrators that are provided by the manufacturer or third party commercial vendors.

The histopathology laboratory, on the other hand, has lagged behind other sections of the clinical laboratory in the implementation of controls and calibrators. There are no IHC or in situ hybridization calibrators. With few exceptions (e.g., HER-2 tests), manufacturers do not provide test controls. Each histopathology laboratory is left to fend for itself in creating, storing, and validating positive controls. Histopathology laboratories typically generate their own controls from tissue specimens that are left over, after a diagnosis has been rendered. Most histopathology laboratories use tissue samples previously documented to contain the particular desired antigen as a positive control. The laboratory documents, sections, and archives a bank of tissues that will serve as controls. As the tissue controls are depleted, new tissues or tumor samples are procured to replace those expended. With laboratories generating their own control tissues, there is little inter-laboratory standardization.

There are significant risks to not having a suitable assay control. For example, a positive IHC test result indicates the presence of the target, such as the HER-2 protein on the membranes of breast cancer cells. A negative IHC test result (the absence of a colored reaction product) can be caused by: (1) the absence of the target (if the assay worked properly), or (2) a failure of the assay itself. In the absence of a positive control, it is difficult or impossible to distinguish between those two possibilities. The consequences of this problem can be severe. For example, a breast cancer specimen that fails to stain for HER-2 as a result of undetected assay failure may deprive a patient of life-extending treatment.

Consequently, there is a need for improved devices and methods for quality control and calibration of assays such as immunohistochemistry and in situ hybridization.

SUMMARY

The invention is set out in the appended set of claims.

In general, a device that can serve as a quality control and/or calibrator, and methods of using the device, for analytes that are often measured in the context of a cell but may include other histologic parameters, is described. Typically, measurements of cellular analytes are performed with the aid of a microscope, by analyzing the intensity of a color or fluorescent light in the spatial context of a cell, often on tissue sections mounted on a glass microscope slide. The device and methods described herein are for the purpose of improving standardization, reproducibility, and accuracy of such measurements. The method can be applicable to the determination of the presence or quantity of an analyte that is measured in the context of a microscopic image, which commonly includes immunohistochemistry and in situ hybridization.

Immunohistochemical asssays are typically performed by the sequential application of various components, such as solutions, solvents, or reagents, to the tissue or cellular sample. Examples of these components include a solvent for removing paraffin wax, a buffer solution for performing antigen retrieval, a primary antibody with an affinity to an analyte, and a detection reagent for converting the primary antibody binding event into a detectable signal.

The method can include the development of a novel embedding medium to retain small beads (e.g., 1-20 micron diameter) or other small particulate matter onto the surface of a microscope slide. The particulate matter can also include cells or other similar biologic materials. The embedding medium can be relatively clear (for example, transparent or translucent) and can directly retain the beads on the slide even after immersion in organic solvents, such as xylene or alcohol. These solvents are used in routine histologic preparation of tissue samples mounted on microscope slides. The term "directly" (for example, as in "directly retains the beads on the slide") means that the embedding medium holds beads on the slide (e.g., in the context of routine histologic processing of tissues and cells) without the need for another subsequent embedding medium, such as paraffin wax. For example, this can differ from the use of agarose as an embedding medium, which typically requires subsequent embedding in paraffin wax for retention of cell lines on a glass microscope slide. In addition, the embedding medium retains the beads on the slide even after immersion in boiling water. Immersion in hot water (for example, buffered to a desired pH) can be required for a procedure commonly known as "antigen retrieval", usually used in clinical laboratories for immunohistochemical staining of tissue samples mounted on microscope slides. In addition to being resilient, the embedding medium is porous, allowing reagents (such as, for example, antibodies or other proteins) to permeate through the sample. This feature enables the performance of assays on the beads for the presence of macromolecules linked to their surfaces. The embedding medium is referred to as a "liquid matrix" or "liquid tissue matrix" because, like a tissue extracellular matrix in a tissue section, it can hold cells and tissue elements (that comprise the tissue) together. The liquid matrix may include components that are found in an extracellular matrix, such as, for example, collagen and protein. An important distinction from an actual extracellular matrix is that the liquid matrix can be dispensed in liquid form before it dries and solidifies. Solidifying can occur spontaneously after dispensing, as the droplet dries, and can be enhanced by warming. The liquid matrix provides for directly adhering small particulate matter to a surface (such as, for example, a microscope glass slide), without the need for subsequent embedding in paraffin wax or microtome sectioning.

The beads (or other particulate matter) can be preferably dispersed in the liquid matrix and of a size that approximates cells, such that they can be readily seen by microscopy. The beads can be coated with one or more types of macromolecules. The macromolecules can be selected so that they produce a reaction in an assay that is used for research or clinical diagnostic purposes. Consequently, the beads with a coated macromolecule can serve as a positive assay control. The macromolecule can preferably be a compound that is still recognized by the assay after exposure to heat (e.g., boiling) and organic solvents (e.g., xylene, alcohol), which are common treatments used in the histologic preparation of tissue. For immunohistochemical assays, the macromolecule can be preferably a peptide, for example, approximately 10 - 30 amino acids long, whose sequence resembles the epitope of an antibody used in the assay. Additionally, if the concentration of macromolecule on the bead is known, then the bead can serve as an assay calibrator. These attributes can be useful in the performance of assays such as immunohistochemistry and in situ hybridization. For example, if the beads are placed on a slide that also bears a patient sample, then the beads can provide a positive assay control. The pathologist viewing the slide can observe the stain intensity of the beads and compare the intensity to an expected stain intensity. The measurement of staining intensity can be quantified by visual estimation or by using photomicroscopy coupled with image analysis software. The expected stain intensity is calculated based on prior measurements, on previous days. If the actual stain intensity is within an acceptable pre-established range, then the pathologist or technologist performing the assay will know that the assay worked properly. For example, a negative test result on a patient sample with an acceptable positive control means that the test result can be accepted as a true negative.

The liquid matrix may comprise several different types of compounds mixed together, including a carbohydrate, protein, solubilized collagen, a non-ionic detergent, or an anti-microbial preservative, or combinations thereof. Examples of suitable carbohydrates can include glycogen, methyl cellulose, or trehalose. Examples of proteins can include keyhole limpet hemocyanin (KLH), bacterial glutathione-S-transferase, or bovine gamma globulin. Examples of collagen can include solubilized type I or type IV collagen. An example of a suitable non-ionic detergent can be beta-octyl glucopyranoside.

The method also optionally can include calculating the molar concentration of macromolecule per bead. A preferred method of this calculation can depend on knowing the molar extinction coefficient of the macromolecule (or a part of the macromolecule) with which the beads are coated. By releasing the macromolecule from the beads into solution and measuring spectrophotometric absorbance, the total molar amount of macromolecule can be calculated. Dividing that amount by the number of beads yields a concentration per bead.

The method also optionally can include evaluating an antigen retrieval protocol and determining if it is functioning properly in reversing the masking effect of formalin fixation. This method includes a description for simulating the effect of formalin fixation on tissue samples by using formalin-fixed peptides immobilized onto beads.

In one aspect, a method of performing an assay can include applying beads containing a quality control moiety and a liquid matrix to a solid surface, wherein the liquid matrix subsequently solidifies and adheres the beads to the solid surface, and processing the solid surface with attached beads and solidified matrix in an assay for the detection of an analyte, wherein processing includes immersing the solid surface with adherent beads and solidified matrix in a liquid greater than 60 degrees Centigrade whereby components of said assay react with the quality control moiety so as to cause a positive result.

In another aspect, an assay device can include beads with an attached quality control moiety, said moiety being reactive in an assay and thereby capable causing a positive test result in said assay and further wherein said assay includes an incubation step at a temperature greater than 60 degrees Centigrade; a liquid matrix in which the beads are suspended, wherein the liquid matrix is liquid at room temperature but solidifies after application to a solid surface and adheres the beads to the solid surface, and further wherein a positive assay reaction occurs when the solid surface with attached beads and matrix are processed in the assay.

In another aspect, an assay device can include a first set of beads with an attached quality control moiety that is reactive in an assay wherein a positive assay reaction produces an optical test result; a color standard bead that, without being processed in an assay, exhibits a positive assay reaction optical test result; and a liquid matrix in which the first set of beads and color standard beads are suspended, wherein the liquid matrix subsequently solidifies after application to a solid surface and retains the beads on the solid surface during processing in the assay. In certain embodiments, the device can include a second set of beads with an attached second quality control moiety that is different than that which is attached to the first set of beads, said second set of beads also being suspended in the liquid matrix.

In certain embodiments, the solid surface can be a microscope slide. In certain embodiments, the liquid matrix can include a carbohydrate and a protein. In certain embodiments, the assay can be an immunohistochemical stain. In certain embodiments, the method can include contacting the solid surface and attached beads with a paraffin wax removing solvent or contacting the solidified matrix and adherent beads with a solution containing a protein cross-linker before processing in the assay. In certain embodiments, the method can include performing the assay on a biological sample that is also mounted on the same surface. In certain embodiments, the method can include measuring the quality control moiety concentration per bead. In certain embodiments, the beads can be of an average length of 1-20 microns. In certain embodiments, the quality control moiety can be covalently linked to the beads. In certain embodiments, the quality control moiety can be a peptide. In certain embodiments, the step at a temperature greater than 60 degrees is antigen retrieval.

In certain embodiments, the positive assay reaction optical test result of the color standard beads can be of approximately the same magnitude as that of the first set of beads. In certain embodiments, the quality control moiety can be covalently attached to the first set of beads. In certain embodiments, the assay can include an incubation of the beads and solidified matrix in a liquid greater than 60 degrees Centigrade. In certain embodiments, the color standard bead can be distinguishable from the first set of beads by virtue of a different physical dimension.

In another aspect, an assay device can include particulate matter having an attached quality control moiety that is reactive in an assay, and a liquid matrix in which the particulate matter is suspended, wherein said liquid matrix is liquid at room temperature but solidifies after application to a solid surface and adheres to the solid surface and by solidifying on the solid surface, the liquid matrix adheres the suspended particulate matter to the solid surface, the liquid matrix withstands heating to 60 degrees Centigrade in an aqueous solution so that at least 25 percent of the particulate matter remains adherent to the solid surface, and is compatible with the assay, in that contacting one or more assay reagents to the surface of the solidified matrix according to the assay protocol causes a positive assay reaction to the adherent particulate matter with attached quality control moiety. In certain embodiments, the solid surface can be transparent. In certain embodiments, the solidified matrix can be immersed in an organic solvent so that at least 25 percent of the particulate matter remains adherent to the solid surface. In certain embodiments, the particulate matter can be less than 100 microns in length, or less than 20 microns in length.

In another aspect, a method of performing an assay can include identifying a quality control moiety that, when present in a sample being tested by an assay, causes a positive assay test result, attaching the quality control moiety to a bead, suspending the bead in a liquid matrix, applying the bead and a liquid matrix to a microscope slide wherein the liquid matrix directly adheres the bead to the slide, processing the bead that is adherent to the microscope slide in said assay wherein a step of the process includes immersion of the bead in a liquid at or higher than 60 degrees Centigrade, and quantifying the magnitude of the positive assay test result.

In certain embodiments, quantifying can be performed manually, by visual estimation. In certain embodiments, the method can include measuring the quality control moiety concentration per bead. In certain embodiments, the method can include contacting the slide with a paraffin wax removing solvent. In certain embodiments, the method can include contacting the bead with a protein and formaldehyde after attaching the quality control moiety to the bead but before suspending it in the liquid matrix.

In another aspect, a method of assay calibration can include attaching a quality control moiety to beads using a cleavable chemical cross-linker, said quality control moiety having an attached fluorochrome with a known spectrophotometric absorptivity, creating a bead suspension in a liquid, cleaving said cleavable chemical cross-linker so as to release the quality control moiety from the beads, so that the released quality control moiety dissolves in the liquid, measuring the spectrophotometric absorption of the quality control moiety dissolved in the liquid, calculating the concentration of the quality control moiety by using the spectrophotometric absorptivity in the calculation, and calculating the number of quality control moieties per bead.

In certain embodiments, the method can include creating a second set of beads with attached quality control moiety, said second set of beads having a different number of quality control moieties per bead. In certain embodiments, the method can include determining the relationship between fluorescence intensity of the beads and the number of quality control moieties per bead. In certain embodiments, the method can include measuring the fluorescence of a third set of beads that has an attached quality control moiety with a fluorochrome, wherein the quality control moiety concentration per bead is initially unknown and, determining the quality control moiety concentration per bead in this third set from the determined fluorescence intensity - quality control moiety concentration relationship. In certain embodiments, the cleavable chemical cross-linker can include a disulfide bond and further wherein the cleaving of said chemical cross-linker comprises exposure to a reducing agent. In certain embodiments, the method can include adhering the beads to a microscope slide and processing the beads in an assay that produces a positive result in the presence of the quality control moiety. In certain embodiments, the method can include processing a biological sample mounted on a microscope slide in the same assay, said biological sample having an analyte that causes a positive result in the assay, measuring the magnitude of the assay result from both the biological sample and the beads, and comparing the two magnitude measurements so as to estimate the analyte concentration in the biological sample. In certain embodiments, the biological sample and the beads can be on the same microscope slide.

In another aspect, a multiplex immunohistochemistry test device can include a first set of beads onto which there is a covalently coupled first peptide, said first peptide causing a positive assay result in a first immunohistochemical assay, and further wherein the first peptide does not cause a positive assay result in a second immunohistochemical assay, a second set of beads onto which there is a covalently coupled second peptide, said second peptide causing a positive assay result in the second immunohistochemical assay, and further wherein the second peptide does not cause a positive assay result in the first immunohistochemical assay, and a liquid matrix in which both said first and second sets of beads are suspended, said matrix having the property of solidifying when dispensed onto the surface of a microscope slide and thereby adhering said beads to the microscope slide even after immersion in a heated liquid at or above 90 degrees Centigrade. In certain embodiments, the device can include a third set of beads that are colored even without processing through an immunohistochemical assay and are of a different physical dimension than the first or second set of beads. In certain embodiments, the first and second immunohistochemical assays can detect the same analyte but at different epitopes.

In another aspect, a method for creating a formaldehyde-fixed quality control can include attaching a peptide to a bead, said peptide having the property of causing a positive assay result in an immunohistochemical assay, said positive result being of a first color intensity, contacting the beads with a protein solution and liquid formaldehyde, and processing the beads in the immunohistochemical assay with a second assay result, having a second color intensity that is diminished relative to the first color intensity.

In certain embodiments, the method can include the step of antigen retrieval, which causes an immunohistochemical assay result to be increased in color intensity relative to the second color intensity. In certain embodiments, the method can include suspending the beads in a liquid matrix to form a bead - liquid matrix suspension and dispensing the bead - liquid matrix suspension onto a microscope slide wherein the liquid matrix directly adheres the beads to the microscope slide surface for subsequent processing in the immunohistochemical assay. In certain embodiments, the method can include, after contacting the beads with a protein solution and liquid formaldehyde, incubating the beads so as to cause evaporation of the liquid formaldehyde.

In another aspect, a solution can include a carbohydrate, a protein, and solubilized collagen wherein the solution solidifies after application to a solid surface so that particles suspended in the solution are adhered to the solid surface.

In certain embodiments, the solution can be characterized by the capacity to retain at least 25% of the particles after solidification and then immersion in a liquid heated to 60 degrees Centigrade or higher. In certain embodiments, the carbohydrate can be glycogen, methyl cellulose, trehalose, chitosan, or an alginate. In certain embodiments, the protein can be keyhole limpet hemocyanin, glutathione-S-transferase, or gamma globulin. In certain embodiments, the collagen can be solubilized type 1 or solubilized type 4 collagen. In certain embodiments, the solution can include a non-ionic detergent. In certain embodiments, the solution can be characterized by the capacity to retain at least 25% of the particles after solidification and then immersion in an organic solvent.

The methods and systems described herein have advantages over prior approaches, for example, cell lines have been tested as substitutes for tissue samples. Formalin-fixed paraffin-embedded cell lines have also been used as positive controls, such as for IHC tests. See, for example, Rhodes, A., et al., Evaluation of HER2/neu immunohistochemical assay sensitivity and scoring on formalin-fixed and paraffin-processed cell lines and breast tumors. Anat. Pathol., 2002. 118: p. 408-417. Rhodes, A., et al., A formalin-fixed, paraffin-processed cell line standard for quality control of immunohistochemical assay of HER-2/neu expression in breast cancer. Amer. J. Clin. Pathol., 2002. 117: p. 81-89. Riera, J., et al., Use of cultured cells as a control for quantitative immunocytochemical analysis of estrogen receptor in breast cancer. Amer. J. Clin. Pathol., 1999. 111: p. 329-335. In theory, cell lines might represent an inexhaustible source of tumor cells expressing a particular analyte. Cell lines can be grown in large batches, formalin-fixed and then dispersed in agar, embedded in paraffin, mounted on paraffin blocks, microtome-sectioned, mounted on microscope slides, and distributed nationally as a commercial product. In this manner, slides bearing formalin-fixed cell lines embedded in paraffin wax can theoretically serve as a standardized positive control. In practice, however, this goal has been difficult to achieve. Commercially available slides bearing cell lines with low and high levels of expression for HER-2 can be purchased. They are often supplied as part of the HER-2 assay kit by commercial vendors. Unfortunately, cell lines do not sufficiently address clinical needs for IHC controls. Exemplary drawbacks include:

1. Cell populations do not have a single level of expression for an analyte. There is heterogeneity in the population. This heterogeneity complicates efforts to standardize and results in lower measurement precision in the control. The level of expression can also drift over time, as the cells are propagated in culture. For example, MCF-7 cultures harvested over a span of 7 weeks produced cultures that ranged from 45-90% ER+ cells. See, Riera, J. et. al., cited previously.

2. They are expensive to produce. Growing the cells is only the beginning. The cells must then be detached from the plastic surface and formalin-fixed. They are then dispersed in an agar matrix, which temporarily holds them in place during subsequent dehydration and permeation with paraffin wax. Treatment with paraffin wax is required, as agar (or agarose) will not suffice in retaining cells on a slide during harsh treatments such as antigen retrieval and immersion in solvents. Rather, the cells are directly adhered to a microscope slide by virtue of the embedding in paraffin wax. After the permeation in wax, the cells are dehydrated and embedded in paraffin blocks, then sectioned with a microtome, and mounted on microscope slides. The labor-intensive aspect of this process cannot be solved through automation, as there is no automated technology for paraffin block embedding and sectioning.

3. They presently exist commercially only for HER-2. There are over 100 other immunostains used for clinical testing.

The methods and systems described herein overcome these disadvantages.

Other aspects, embodiments, and features will be apparent from the following description, the drawings, and the claims.

BRIEF DESCRIPTION OF THE DRAWINGS

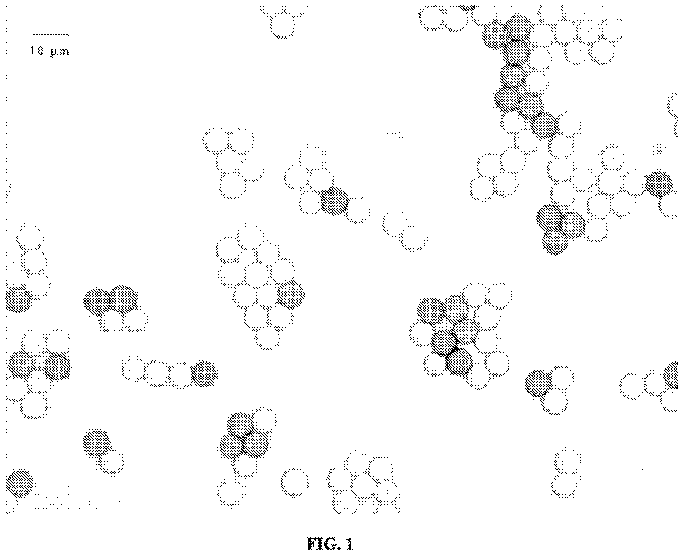

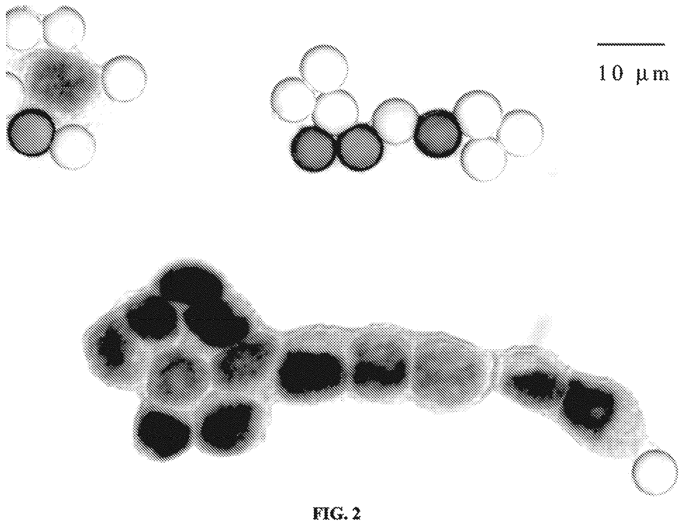

FIG. 1 represents beads embedded in matrix, after an immunohistochemical stain for human estrogen receptor, demonstrating that some beads (bearing the relevant peptide) stain while others (bearing an irrelevant peptide) do not. FIG. 2 represents MCF-7 cells embedded in the liquid matrix, after immunohistochemical staining for human estrogen receptor. The cells demonstrate nuclear staining, as expected, since the estrogen receptor is primarily localized to the cell nucleus. The figure also includes beads, some of which have the immunoreactive peptide (brown stain) while others have an irrelevant peptide (no stain).

FIGS. 3A and 3B represent calibration curves of HER-2, using beads of known molar concentration.

FIGS. 4A and 4B represent formalin fixation of peptides on beads, showing dependence on antigen retrieval.

FIG. 5 represents unstained (left side, denoted "A") and stained (right side, denoted "B") cocktail of test and color standard beads. The color standard beads are smaller and colored brown, regardless of the stain. The test beads are a mixture of four different beads, each bearing a different quality control moiety. The test beads that become colored after staining are those that react with the stain. Test beads with an unrelated quality control moiety are left unstained and represent a negative control.

FIG. 6 represents representative example of a 3+ stain intensity on a cocktail of test and color standard beads.
FIG. 7 represents representative example of a 2+ stain intensity on a cocktail of test and color standard beads.
FIG. 8 represents representative example of a 1+ stain intensity on a cocktail of test and color standard beads.

## DETAILED DESCRIPTION

Definitions

Quality control moiety. For purposes of clarity, "quality control moiety" is defined as the analyte such as it exists in cells or tissue sections, a recombinant protein, analyte fragment, or analyte mimic (e.g., mimotope) that causes the detectable reaction as part of this herein described quality control or calibrator device. The analyte fragment may be an enzymatically cleaved protein fragment derived from the analyte. Alternatively, the quality control moiety may be a synthetic peptide that is similar to the primary amino acid sequence from a portion of the analyte. For example, the quality control moiety for an immunohistochemical assay specific for the cell surface glycoprotein HER-2 may include the HER-2 glycoprotein, a fragment of HER-2 such as the extracellular domain, or a synthetic peptide that represents or mimics the anti-HER-2 antibody epitope. There will usually be different quality control moieties for each assay, reflecting the particular protein or nucleic acid (or other) specificity of the assay. For example, each primary antibody in an immunohistochemical assay will usually have its own quality control moiety. An exception to this will be if two primary antibodies, both specific to the same protein, bind to epitopes that are identical or adjacent to each other. In such a circumstance, a single quality control moiety could be suitable for both primary antibodies.

Moreover, it is intended that a non-protein may also serve as a quality control moiety, depending on the nature of the analyte. For example, instead of an immunohistochemical stain, the same principles also apply to in situ hybridization (ISH), which uses a nucleic acid probe instead of an antibody. For ISH, the quality control moiety could represent the cDNA associated with a portion or all of a particular gene. Alternatively, the quality control moiety could represent a short stretch of DNA, such as an oligomer. An analyte mimic in this context might represent a nucleic acid aptamer that binds to a particular ISH probe. Any molecule that binds to a nucleic acid probe (for example, for ISH) can represent the quality control moiety.

Biological sample. The term "biological sample" can be any cell-containing sample. For example, the sample can be tissue, blood, urine, cerebral spinal fluid (CSF), sputum, semen, cervicovaginal swab, or intestinal wash.

Assay terminology. The target molecule (analyte) to be detected in an assay or stain is also sometimes referred to as the "antigen". As used herein, the term "antigen" means a molecule detected by an antibody. The term analyte is broader, being not limited to assays with antibody detection systems. The term "immunocytochemical" is used synonymously with "immunohistochemical", both referring to an antibody test for in situ identification of analytes in cells or in a tissue section.

For immunohistochemistry, the typical target molecule (e.g., the "analyte") is a protein, glycoprotein, or lipoprotein that is detectable by antibody binding. Other analytes associated with other types of histochemical stains may be carbohydrates, lipids, or proteins, or combinations thereof. Representative histochemical stains include the periodic acid-schiff stain, mucicarmine stain, or reticulin stain. Analytes can also be nucleic acids (e.g., that are detectable by in situ hybridization techniques). For ISH, the target molecule is usually a nucleic acid detected by a nucleic acid probe complementary to the nucleotide sequence of the target molecule. Various features of the technology will be applicable to histochemical stains and in situ hybridization.

For application to immunohistochemistry, the quality control moiety is detectable by a specific antibody, referred to herein as the primary antibody. The primary antibodies are typically monoclonal but may also be polyclonal. In order to simplify manufacture, it is desirable to avoid the need for purification of the many different analytes that are used in clinical diagnosis. Therefore, a convenient form of the quality control moiety of the device described herein is a synthetic peptide that represents or mimics the analyte to be detected on cells or tissue sections, and specifically binds to the primary antibody under substantially the same conditions as the analyte to be detected.

In the description, procedures (also known as "protocols") are described that are involved in processing an assay, such as an immunohistochemical stain. The term "assay" is generally a more generic term than "stain", since there are many other types of assays besides stains of biologic samples. Immunohistochemistry is a type of immunoassay. It is also a type of "stain". Assays are comprised of one or several steps whereby an assay reagent is added to a test sample. Often, the first reagent is removed by rinsing (or "washing") and a second reagent is added. Many assays involve adding a series of sequential assay reagents, with each reagent being incubated for a defined period of time according to an assay protocol. Another assay, in situ hybridization (ISH), is a nucleic acid assay and is also often referred to as an ISH "stain". A histochemical assay is a chemical assay on a tissue sample. In this patent specification, the terms "assay" and "stain" are used interchangeably. Even though "stain" may be specified in this patent application because it is often used by convention, the invention is not necessarily limited to assays on tissue or cellular samples. As used herein, the term "processing" an assay means performing the steps of an assay required to detect the presence, or absence, of the analyte. For example, "processing" or "staining" can mean (if used in the context of immunohistochemistry) performing the steps of an immunohistochemical stain so as to detect the presence of an analyte. In this patent application, the stain or assay can also include preparative steps such as deparaffinization, rehydration, and antigen retrieval. Antigen retrieval is a process whereby biological samples can be heated in an aqueous liquid, usually buffered to a specific pH. The temperature can be raised to at least 90 degrees Centigrade. More commonly, the liquid temperature is raised close to, at, or beyond, boiling. To raise the temperature beyond boiling, the samples can be placed inside a pressure cooker. The term "processing" an immunohistochemical assay or "staining" also includes contacting the analyte (in the biological sample) with an antibody that specifically binds to the analyte. An antibody can be an example of a biological macromolecule, often used in diagnostic assays.

Both immunohistochemistry and in situ hybridization involve the application (to the biological sample) of reagents that are biological macromolecules. These reagents are applied to the biological sample so that a reaction might occur if the analyte is present. Such biological macromolecules include proteins, such as enzymes and antibodies. In addition, the phrase "biological macromolecules" includes nucleic acids, such as a DNA or RNA probe.

In all assays, the assay result is ultimately determined in a measuring step, at the end of the assay. The specific measuring step employed depends on the assay, but can include a measure of color intensity, fluorescence intensity, light intensity (e.g., chemiluminescence), number of colored spots (such as for HER-2 in situ hybridization), and/or the morphologic appearance of a tissue stain. For example, it includes detecting an antibody bound to a target (e.g., by detecting a colorimetric signal) wherein detection of the signal is indicative of the presence of the analyte (target), and lack of signal detection is indicative of the absence of the analyte.

Peptide A peptides can be conveniently used as a quality control moiety. A peptide is a short chain of amino acid monomers linked by peptide (amide) bonds, preferably 10 - 30 amino acids long. This length provides for both the antibody epitope and a spacer for linkage to a solid surface. Conventionally, peptides longer than 50 amino acids long are termed "proteins".

Challenges in IHC quality control

For most types of clinical laboratory testing, a QC product is simple. For example, a commercial QC product for serum glucose testing is a glucose solution with stabilizers and preservatives. Such controls can be commercially prepared in large quantities for distribution to thousands of clinical laboratories. However, developing standardized QC products for IHC testing, performed on tissue biopsies, has been challenging. Years ago, the National Institute of Standards and Technology (NIST) and the College of American Pathologists (CAP) urged the development of a Her-2 immunostaining standard, but no standard ever emerged. See, Hammond, M., et al., Standard reference material for Her2 testing: Report of a National Institute of Standards and Technology-sponsored consensus workshop. Appl. Immunohistochem. & Mol. Morphol., 2003. 11(2): p. 103-106. Obtaining human tissues with a defined amount of analyte ("analyte": the molecule being measured), in large quantities, is difficult or impossible.

A first problem with the present practice, using leftover tissue samples as controls, is lack of standardization. The use of tissue controls, as described above, necessarily means that the analyte concentration varies both within and between labs. Variation in analyte concentration is due to intrinsic differences in the concentration of analyte in tissues obtained from different individuals, effects of long-term storage, and effects of pre-analytic processing. Examples of pre-analytic processing variables include the type and length of fixation, time before immersion in fixative, and the details of immersion in solvents and molten paraffin. Even amongst the cells of a single tumor, there is variability in the analyte concentration (per cell). Because of this heterogeneity, tissue controls can be used to detect gross assay failure but are less optimal for monitoring gradual loss in assay sensitivity. This is a serious limitation. Many markers that are frequently used to help identify the tissue origin of tumors are weakly expressed in high-grade (the most aggressive and dangerous) cancers. In contrast, positive control tissues are often chosen because they express these antigenic markers strongly. In this setting, degradation of assay performance will not be readily apparent since the positive control tissue continues to stain with an intensity that appears to be appropriate (as human eyes are not precise gauges of optical color intensity). However, a weakly expressing tumor from a patient may not stain at all, thus resulting in a false negative interpretation in spite of having a positive control on the slide.

Another problem with the use of leftover tissue samples as controls is that their use is labor-intensive. There is effort (and cost) in frequently and repeatedly screening archival (leftover) tissue samples, retrieving the tissue blocks from storage, re-testing the block to ensure positivity, and finally cutting the paraffin sections and mounting them on glass microscope slides. These costs are less easily captured (for example, in an accounting) than the cost of purchasing a control from an outside vendor, but nevertheless represent a real expense to the lab.

The relatively high cost of obtaining positive control slides usually causes laboratory staff to use the control as a "batch" control. A batch control means that a single positive control slide is used to monitor the successful completion of a batch of slides stained at the same time. The use of batch controls, though common, is sub-optimal because the failure of any given slide can be independent of its neighbors. A batch control slide may stain perfectly while its batch-mate may fail to stain due to any of a number of instrument failure modes. Examples include missed or insufficient reagent additions, failure to wash properly between reagent additions (e.g., running out of wash solution), failure to remove wash solution prior to the next reagent addition, individual slide heater failure, and others. If the batch control is positive, then such failures will likely go undetected. Consequently, the tissue would be interpreted as not expressing the target of interest when, in fact, it does - - a false negative. It is desirable that every slide will have a positive control to verify stain performance.

Purified analyte as an IHC control. Theoretically, an alternative approach for developing external positive controls would be to place the purified analyte on a microscope slide. This concept differs from previously described methods because it avoids the use of biological cells or tissues. Rather than mounting fixed cells or tissue sections on a slide as a test control, this alternative envisions the placement of the purified target molecule (the "analyte"). The development of a colored reaction product (such as in an IHC stain) would be proportional to the concentration of the analyte, at least within the linear range of the assay. By placing an analyte on a glass microscope slide, the intensity of the colored reaction product can be assessed as a quality control check.

Unfortunately, the use of purified proteins as IHC controls does not adequately address clinical needs. For example, most analytes that are relevant in the clinical practice of immunohistochemistry are large complex glycoproteins, found in cells at low concentrations. Consequently, very few are readily available in purified form. Recombinant DNA production methods potentially provide an additional source of analytes, but at a price that is not commercially feasible for a quality control or calibrator product. Even if the analytes were available at a suitable cost, protein stability is an additional difficult hurdle to commercialization. These proteins need to withstand immersion in organic solvents (e.g., alcohol, xylene, or xylene substitutes), for deparaffinization, and still retain the ability to be recognized and capable of binding to an antibody. Xylene and certain other solvents known as xylene substitutes are capable of dissolving paraffin wax. These solvents typically cause protein denaturation. In addition, the proteins need to withstand immersion in boiling water (or near-boiling temperatures, depending on the analyte), for antigen retrieval, another highly denaturing process. Few proteins can meet these stability requirements.

Peptides as IHC controls A variant of using the purified analyte (as an immunohistochemistry quality control) is the use of peptides. See, for example, U.S. Patent No. 7,011,940B 1. Peptides that represent the primary antibody epitope and bind the antibody with comparable affinity as the native protein can act as a surrogate analyte for the purposes of quality control and calibration. Short peptides representing the IHC primary antibody epitope can serve as excellent analytes in lieu of the complete (intact, full length) protein analyte (as it exists in cell and tissue sections). Usually, the peptide control is similar or identical to the epitope linear sequence as found in the native protein. It is also feasible to use peptides that bear little resemblance to the linear amino acid sequence of the native epitope. Such peptides still bind to the antigen binding region of the antibody, in a specific fashion, even though their amino acid sequence is different than that found in the native protein. These peptides are often referred to as "mimotopes".

Peptides selected as quality control moieties can be derived by screening a random combinatorial peptide library expressed in M13 bacteriophage. See, for example, Sompuram, S, V Kodela, H Ramanathan, C Wescott, G Radcliffe, & SA Bogen. 2002. Synthetic peptides identified from phage-displayed combinatorial libraries as immunodiagnostic assay surrogate quality control targets. Clin. Chem. 48(3):410-420. The peptides identified from this screen are specific for binding only to the desired analyte-specific antibody. In addition, the peptides are relatively inexpensive, readily detected in an IHC stain, can tolerate immersion in solvents and boiling water, and are stable upon storage. See, for example, Sompuram, S, V Kodela, K Zhang, H Ramanathan, G Radcliffe, P Falb, & SA Bogen. 2002. A novel quality control slide for quantitative immunohistochemistry testing. J. Histochem. Cytochem. 50:1425-34.

Peptides can be attached directly to glass microscope slides. Upon IHC staining, a 2-3 mm diameter colored spot appeared. By attaching a consistent, reproducible amount of peptide to every glass slide, the analyte concentration could be standardized. The peptides on glass slides were capable of serving as quality control devices, in that the intensity of the colored spot could be correlated with the efficacy of the stain. The fact that the quality control device was printed on the microscope slide had certain drawbacks, however, relating to cost of manufacture, customer convenience, and adaptability with conventional image analysis software.

In order to overcome these limitations, an improved method was developed for performing quality control and calibration for these advanced types of histopathology stains.

Beads as controls

The improvements associated with the present invention includes a quality control and/or calibrator device comprising one, or more, quality control moieties attached to a small bead. Preferably, the beads are of the approximate size of a cell of interest. By definition, beads are not biological cells. Rather, beads are comprised of a material that is relatively inert or non-reactive in an assay in which they are used, and intended for examination using optical magnification. This last parameter implies that beads are less than 1 millimeter in length. Preferably, beads are of a comparable size to cells (for example, average size of 1 - 20 microns in the longest dimension). Although beads are a convenient material, any small particulate matter of a similar size (average size of 1 - 20 microns in the longest dimension) can be used in place of a bead. Like cells, beads are often round, although a round shape is not required. If the bead is used as a quality control or calibrator, then preferably the bead is evaluated by the same type of microscopy that is used for the assay sample itself. Bead size is preferably uniform (for example, from bead to bead) but uniformity of shape is not required. Thus, the term "bead" can mean any small particulate non-cellular object that fulfills these aforementioned functional specifications.

For example, the beads can be made of polystyrene or glass. In order to attach quality control moieties, the polystyrene beads can derivatized with functional groups, such as amine or carboxyl groups. A commercially available example is Polybead Carboxylate, from Polysciences Inc., Warrington PA. Those beads are monodisperse polystyrene microspheres that contain surface carboxyl groups, facilitating covalent binding of proteins to the bead surface through a cross-linking reaction. Alternatively, glass beads can be used. A commercially available example is Monodisperse Silica Microspheres (7.8 average micron diameter) from Cospheric, Santa Barbara, CA.

Covalent attachment of quality control moieties can be beneficial for immunohistochemistry. By covalent attachment, it is meant that there is a continuous series of covalent chemical bonds linking the bead (or other particulate matter) to the quality control moiety. Non-covalent attachment would be suitable for many other types of assays, such as immunoassays. However, immunohistochemistry typically requires immersion in a boiling (or near-boiling) aqueous buffer for antigen retrieval as well as immersion in organic solvents (for deparaffinization and re-hydration). In situ hybridization also entails high temperature treatments for melting and annealing of DNA strands, often above 60 degrees C. Without covalent attachment, these treatments will usually dissociate the quality control moiety from the bead. Consequently, a feature of the present approach is that the chemical attachment will withstand these temperatures.

Coupling the quality control moiety to the bead (example) A first step in attaching the quality control moiety to the bead is the initial attachment of silane that has one or more free amine groups. The amines can then serve as an attachment point in a subsequent cross-linking to the quality control moiety. The attachment of aminosilane to the bead involves preparing the bead surface by cleaning and etching in acid. After rinsing out the acid, the beads are then incubated with a commercially available silane solution (e.g., 3-aminopropyltriethoxysilane). The unattached silane is then rinsed off with a solvent for the unattached silane (e.g., with acetone) and ready to use in a cross-linking reaction with the quality control moiety.

The free amine group on the aminosilane can then be converted to a chemically reactive group causing a covalent linkage to the quality control moiety. The nature of the chemically reactive group that is formed depends on the type of cross-linker that is used. For example, if 1,1'-carbonyl di-imadazole (CDI) is used, then an isocyanate reactive group is formed. Exemplary cross-linking chemistry is described, for example, in U.S. Patent No. 6855490.

Instead of CDI, an alternative cross-linker is dimethyl suberimidate-2HCl (Pierce Chemical Co., Rockford, IL), which, upon reaction with the aminosilane, generates a reactive imidoester group. Both the isocyanate or imidoester groups can form a covalent bond to free amine groups on a quality control moiety. Another suitable cross-linker is Bis[sulfosuccinimidyl] suberate, abbreviated "BS3", available from Pierce Chemical Co., Rockford, IL. BS3 contains an amine-reactive N-hydroxysulfosuccinimide (NHS) ester at each end of an 8-carbon spacer arm. NHS esters react with primary amines at pH 7-9 to form stable amide bonds (a type of covalent bond). In fact, any chemical cross-linker can potentially be used if it is capable of creating a covalent link between the glass beads and the quality control moiety.

The conversion of the free amine (on the aminosilane) to a chemically reactive group can be accomplished in either in a one or two-step process. In a two-step (sequential) process, the aminosilane is first converted to a chemically reactive group. The cross-linking reagent is then rinsed out from the solution and the quality control moiety is then added. In this way, the chemically reactive groups on the beads (associated with the aminosilane molecules) can form a covalent bond to the quality control molecule. In a one-step process, the quality control moiety is added at the same time as the cross-linker. An advantage of the two-step reaction is that cross-linking of two quality control moieties to each other is avoided. Linking two quality control moieties to each other is an undesirable side reaction that has the potential to diminish the amount of quality control moiety that binds to the bead. Moreover, it diminishes the concentration of cross-linker in solution that can link the quality control moiety to the bead. However, a two-step reaction is not without its drawbacks. A disadvantage of many two-step reactions is that the chemically reactive group on the bead (or other particulate matter) may undergo hydrolysis or other type of degradation, depending on the cross-linker used, before the quality control moiety can be added. As a result, fewer quality control moieties will bind to the beads. In experiments, BS3 was shown to be best used in a one-step reaction with aminosilane-coated beads and the quality control moiety.

Epitope amplification by polymerization (example) Within the linear range of the assay, the signal intensity in an immunohistochemical assay can be expected to be approximately proportional to the number of antibody binding sites on the bead. Consequently, it may be possible to extend the upper end of the linear assay range, generating more intensely staining beads, by increasing the number of peptide epitopes attached to the bead. Preferably, the peptides will be spaced from one another, so as to minimize steric interference of one bound antibody to the next. To accomplish this goal, a method of polymerizing the peptide on the bead surface, forming long strands is described. Antibodies can bind anywhere along the length of the strand, which is comprised of repeating peptide epitopes. To synthesize such strands, peptide is coupled to the bead surface as previously described, but with one important change - - the peptides now have a free carboxyl group. In the previously described method, peptides with amidated carboxyl groups were used, rendering them non-reactive and stable. The formation of polymers requires that such amidation is not used.

The first cross-linking (of peptide to the bead) is as previously described, using a cross-linking reagent specific for two free amine groups. The free amine on the aminosilane is linked to the free epsilon amine on a lysine, towards one end of the peptide. As a side reaction, peptides may also link to each other, forming peptide dimers. However, apart from consuming peptide, this side reaction is of no consequence because the peptide dimers and unreacted peptide (monomers) are washed away after the reaction is completed. At the end of the first step, a single peptide (having a free carboxyl group) is attached to the aminosilanes on the bead.

To create peptide polymers, a new cross-linker is added, along with additional soluble peptide (also having both a free amine and carboxyl group). The new cross-linker is one that can link a free amine to a free carboxyl group. For example, the cross-linker 1-Ethyl-3-(3-dimethylaminopropyl)carbodiimide hydrochloride, is added to the peptide-coated beads at 1.0 - 10 mg/mL in a 0.9% sodium chloride, 20 mM MES buffer (4-morpholinoethanesulfonic acid), pH 6. The peptide concentration may be in the range of 0.01 - 10 mg/mL. In this second cross-linking reaction, the free carboxyl group on the bead-immobilized peptide is linked to a free amine on a newly added, soluble peptide, adding a second peptide to the first. That new peptide has, in turn, a free carboxyl group, available for additional cross-linking. This cross-linking continues to form a peptide polymer, until the reaction mixture is exhausted or it is stopped by diluting out or rinsing out the reactants. In this manner, end-to-end peptide polymers form, representing a repeating chain of antibody epitopes. The length of polymerization can be adjusted through varying peptide concentrations and reaction time or temperature.

Methods to identify quality control moieties

_Disadvantages of using whole (native) proteins as quality control moieties_ There are several possible methods for identifying a suitable quality control moiety when the analyte is a protein. Theoretically, the easiest would be to use the analyte itself, such as the whole protein. As previously described, most clinically useful analytes are not readily available at a suitable price for a commercial product of this nature. Moreover, proteins are often not sufficiently stable in the face of solvents and boiling, which are typical treatments for formalin-fixed tissue sections. If the control is placed alongside a tissue sample on a microscope slide, then the quality control moiety must be able to withstand the same treatments. Peptides that represent the primary antibody epitope meet these criteria; they are inexpensive to produce and chemically more stable than many whole proteins.

_Random combinatorial peptide library screening by phage display_ Suitable peptide epitopes can be identified from a random combinatorial library of peptides, expressed in M13 phage. The technology whereby peptides are expressed on M13 phage is often termed "phage display". Methods for identifying suitable peptides from such phage-displayed libraries using biopanning are described, for example, in Sompuram, S, V Kodela, H Ramanathan, C Wescott, G Radcliffe, & SA Bogen. 2002. Synthetic peptides identified from phage-displayed combinatorial libraries as immunodiagnostic assay surrogate quality control targets. Clinical Chemistry 48(3):410-420. An advantage of this approach is that peptides will be identified even if the actual epitope is conformationally-dependent. Peptides that resemble the conformationally-dependent epitope can bind to the primary antibody (that is used in biopanning) and are amplified by multiple rounds of biopanning.

_Epitope identification by overlapping peptides_ An additional method for identifying peptides (to serve as quality control moieties) is by testing peptides that are identical to short stretches of the native protein. If the amino acid sequence of the protein analyte is known, then short peptides based on the sequence can be chemically synthesized. The overlapping peptides approach involves synthesizing a series of peptides that comprise the amino acids of the native protein. For example, the first peptide might be identical to amino acids 1 - 10. A second peptide might be identical to amino acids 4 - 14. A third peptide would then be identical to amino acids 8 - 18, and so on through the length of the protein. In this way, the entire protein can be synthesized as a series of short peptides. To identify the antibody epitope, the peptides are incorporated into an immunoassay, usually by attachment to a solid surface, such as a microtiter well. Each peptide is then individually tested for immunoreactivity to the primary antibody. The epitope is identified by determining which peptides bind to the primary antibody. Since the peptide sequences overlap one another, the primary antibody may bind to 2 or more adjacent peptides expressing overlapping amino acid sequences. This method of epitope identification can be effective if the epitope is a linear epitope, as is generally the case for antibodies that are used for clinical diagnosis. See, for example, Sompuram SR, K Vani, LJ Hafer, & SA Bogen. 2006. Antibodies Immunoreactive With Formalin-Fixed Tissue Antigens Recognize Linear Protein Epitopes. Am. J. Clin. Pathol. 125: 82-90.

_Peptide modifications_ Once the epitope is identified, the amino terminus is preferably acetylated and the carboxy terminus is preferably amidated. Capping of the peptide ends in this way helps improve peptide stability. In addition, a lysine can be inserted at one of the ends to provide for binding to the particulate matter (e.g., bead) through its epsilon amine group. Preferably, a second lysine can be inserted nearby, often spaced from the terminal lysine with a glycine.

This second lysine can have an attached fluorochrome to its side chain. These inserted amino acids are added distant to the end of the peptide bearing the antibody epitope. The amount of peptide to be used in a coupling reaction is often approximately 1-10 mg/mL, but depends on the number of beads. The optimal concentration is typically determined empirically, after experimentation. As an example, 4 mg of peptide can be suitable for binding to 330 mg of beads.

Exemplary embodiments

Bead cocktail for one analyte To increase the utility of the quality control device, beads with different quality control moieties that all relate to the same analyte can be combined into a single product. For example, there are several widely used primary antibodies for the analyte HER-2. The designations of these antibodies are SP3, CB11, 4B5, and Herceptest, the last being a polyclonal antibody preparation. Each antibody is typically sold by a different commercial vendor. The epitopes for the CB11, 4B5, and Herceptest antibodies are identical or immediately adjacent to one another, and therefore can be all accounted for by a single peptide. See, for example, A-S Schrohl, H.C. Pedersen, S.S. Jensen, et. al. Human epidermal growth factor receptor 2 (HER2) immunoreactivity: specificity of three pharmacodiagnostic antibodies. Histopathology 2011 59:975-983. The SP3 antibody epitope is different.

In one embodiment, one would couple only a single type of peptide to beads. For example, a first peptide that is immunoreactive with the 4B5, CB11, and Herceptest antibodies is coupled to a first set of beads. A second peptide that is immunoreactive with the SP3 antibody is coupled to a second set of beads. These different beads can then be mixed together in a final product that is suitable for all of the major HER-2 antibodies. After IHC staining of the bead mixture with a single HER-2 primary antibody, only those beads that bear the relevant quality control moiety for that particular primary antibody will generate a positive reaction. Other beads, such as those bearing peptide epitopes specific for other HER-2 antibodies, will be unstained. These irrelevant beads (having a quality control moiety that is not bound by the primary antibody) serve as a specificity control. In this way, customers have a built-in positive and negative control within the same product. In measuring the stain intensity of the stained beads, the unstained beads can serve as a negative control, helping to establish a baseline (background) color intensity. An example of such a mixture of beads, stained for an antibody to the human estrogen receptor, is shown in FIG. 1. These beads were embedded in a liquid matrix and dispensed onto a microscope slide, to which they adhered. The colored beads have an attached peptide that is immunoreactive with the primary antibody used in the immunohistochemical stain (assay). The uncolored beads have an attached peptide that is not immunoreactive with the primary antibody used in the immunohistochemical stain (assay).

Bead cocktail for multiple analytes In an alternative embodiment, beads bearing different quality control moieties are combined, but the quality control moieties do not all relate to the same analyte. For example, the analytes HER-2, human estrogen receptor (ER), and human progesterone receptor (PR) all relate to breast cancer testing. Therefore, an alternative exemplary embodiment is to combine beads bearing quality control moieties to all three into a single breast cancer quality control product. In this example, beads bearing quality control moieties to different analytes are mixed. Although the analytes are different, they all relate to the same diagnostic question, such as the appropriate treatment for a particular patient's breast cancer.

Anti-immunoglobulin-coated beads In another embodiment, the quality control moiety is an anti-immunoglobulin antibody that binds to the primary antibody. For example, the quality control moiety can be an anti-mouse immunoglobulin (IgG) antibody if the primary antibody is a mouse IgG antibody. In this embodiment, the quality control device monitors for the presence of primary antibody regardless of its ability to bind to an analyte. Moreover, it can monitor whether subsequent immunocytochemical reagents (e.g., secondary antibody or enzyme conjugate) are applied in the correct sequential order. This particular embodiment is not as informative as quality control moieties that bind to a primary antibody through an antigen-combining site, such as peptides, but may be helpful for certain quality control purposes. Using an anti-immunoglobulin antibody as the quality control moiety has the drawback that it may likely be denatured by the deparaffinization and/or antigen retrieval treatments. An alternative quality control moiety with a similar binding specificity for immunoglobulins is to use a peptide with a similar binding specificity. See, for example, T Sugita, M Katayama, M Okochi, R Kato, T Ichihara, H Honda. Screening of peptide ligands that bind to the Fc region of IgG using peptide array and its application to affinity purification of antibody. Biochem Eng J 2013 79:33-40. Advantageously, peptides are more resistant to these denaturing treatments. Other alternative quality control moieties with a similar immunoglobulin binding specificity are Protein A, Protein G, or a recombinant chimera of the two. In fact, any binder with a specificity to immunoglobulins can potentially serve as this type of quality control moiety.

Immunoglobulin-coated beads In another embodiment, the quality control moiety is comprised of immunoglobulins. These immunoglobulins can serve as a target for the secondary (detecting) reagent that is commonly used in immunohistochemical assays. For example, normal mouse or rabbit immunoglobulins would be recognized by a secondary reagent comprising anti-mouse or anti-rabbit IgG antibody. The presence of normal mouse or rabbit immunoglobulins on the beads would therefore test the proper performance and sensitivity of the detection kit components in an immunohistochemical assay.

Liquid matrix

Purpose of liquid matrix Using beads as staining controls requires a method to keep the beads on the glass microscope slide during the staining process. The staining process typically involves the repeated application, incubation, and rinsing of reagents and wash buffers onto the microscope slide bearing a tissue sample and/or the quality control beads. Usually, a series of sequential reagent applications and rinses are required. Without a method for retaining the beads on the glass slide, the beads would be rinsed off. This need is addressed by suspending or covering the beads in a new liquid matrix.

Use and characteristics of liquid matrix Before staining, a small drop of the liquid matrix with suspended microbeads is dispensed onto the glass slide. Typically, this drop is approximately 1 - 3 microliters in volume. Initially, the liquid matrix is liquid at room temperature (20 - 23 C) or in the refrigerator (4 - 8 C). The beads may be suspended in the liquid matrix or, alternatively, dispensed first and then covered by the liquid matrix. Either way, the beads are suspended, and ultimately become embedded in, the liquid matrix. After dispensing the drop of liquid matrix onto a surface, such as a microscope glass slide, the liquid matrix dries to form a porous transparent or translucent solid film, entrapping the beads and adhering them to the slide. Drying the liquid matrix involves leaving it at room temperature so that the water in the matrix evaporates. Alternatively, drying of the liquid matrix can be enhanced by warming the slides, which is often done anyway for routine histology preparation in a process called "baking" the slides. Baking slides serves the purpose of improving tissue section adhesion to the glass slides.

Once dried, the matrix in which the beads are suspended can withstand boiling (in an aqueous buffer) for at least 30 minutes (as is commonly performed for antigen retrieval). Heating of slides is also performed for in situ hybridization, for DNA melting and/or annealing. The exact temperatures depend on the probe and target, but are typically equal to or greater than 60 degrees Centigrade. Also, the matrix can withstand immersion in alcohol and xylene, for deparaffinization. Typically, immersion in these paraffin wax solvents lasts 2-20 minutes. These treatments are commonly used in histologic preparation of tissue sections mounted on microscope slides.

Summarizing, the liquid matrix is initially in a liquid state at room temperature (20 - 23 C) or in the refrigerator (4 - 8 C). Dispensing a small aliquot of the liquid matrix and exposing it to room air allows it to dry out, resulting in its conversion to a solid film. In the solidified (hardened) state, the liquid matrix resists the types of treatments that are commonly used in histology, such as heating and immersion in organic solvents like alcohol (for dehydration) and xylene or xylene substitutes (for deparaffinization).

Distinction from other adhesives The liquid matrix is porous, permitting antibody and reagent penetration. This is an important distinction from adhesives such as cyanoacrylate, which are not porous to biological macromolecules. Consequently, the beads can be contacted by the staining reagents even if they are embedded within the matrix. This feature is termed as being "compatible" with an assay, such as immunohistochemistry or in situ hybridization. With a porous matrix, antibodies, enzymes, enzyme conjugates, enzyme substrates, and other macromolecules are all able to penetrate through. Excess, unused, or unbound reagent can also be washed out after incubation, prior to adding a subsequent reagent. A determination as to whether a matrix is compatible in this way can be empirically determined, by assessing whether cells, beads or other particulate matter, embedded in the matrix, can be stained. If yes (they are stained), then it can be inferred that the staining reagents diffused through pores in the matrix. The fact that assays can be successfully performed on embedded cells, beads, or other particulate matter, resulting in positive test results for reactive quality control moieties and negative results for non-reactive quality control moieties embedded in the liquid matrix, demonstrates that the matrix is porous and therefore compatible with a particular assay.

The liquid matrix characteristics are also different than those of an agarose solution, which has been used by others as a cell embedding medium. An agarose solution is unsuitable as a matrix because it is typically solid at room temperature (20 - 23 C) and liquefies upon warming. If dried as a film onto a glass slide, agarose will dissolve during a procedure such as antigen retrieval, which involves immersion in a hot aqueous buffer. This renders it unsuitable for retaining beads on a slide. This is the opposite of the liquid matrix, which is initially liquid at room temperature and solidifies upon warming (as it dries out). When immersed in a hot aqueous buffer, the solidified matrix does not dissolve. To overcome this limitation, previous descriptions on the use of agarose as a cell embedding medium for immunohistochemistry testing also include a subsequent embedding of the agarose block in paraffin wax, a step that is not necessary for the present invention. Embedding in paraffin wax also necessitates subsequent microtomy into thin sections, which is unnecessary when using the liquid matrix. Similar limitations have also been described for alginate matrices. See, for example, King S.M., Quartuccio S., Hilliard T.S., Inoue K., Burdette J.E. (2011). Alginate Hydrogels for Three-Dimensional Organ Culture of Ovaries and Oviducts. JoVE. 52.

http://www.jove.com/details.php?id=2804, doi: 10.3791/2804, page 2; S-R Shi, C Liu, J Perez and CR Taylor Protein-embedding Technique: A Potential Approach to Standardization of Immunohistochemistry for Formalin-fixed, Paraffin-embedded Tissue Sections, J Histochem Cytochem 2005 53: 1168.

Use with varied particulate matter The liquid matrix can attach many types of small particulate objects to microscope slides, not only beads. For example, the liquid matrix was used to attach dispersed fixed cells, such as tumor cells. FIG. 2 shows an example of fixed tumor cells (MCF-7 cell line) from a malignant cell line, grown in vitro, and then embedded in the liquid matrix and stained with an immunohistochemical stain for the human estrogen receptor. In this particular figure, there are also beads co-mixed in the embedding medium, some of which bear the relevant quality control moiety for this particular immunohistochemical stain. Consequently, some of the beads are stained.

The liquid matrix is simpler and less labor-intensive as compared to the current state of the art for attaching fixed cells to a surface, such as a microscope slide. To get fixed cells to adhere to a microscope slide, the cells are first embedded in agarose. The agarose block is then processed through increasing grades of alcohol, transferred into xylene (or xylene substitutes), and finally molten paraffin, ultimately embedding the cell block in paraffin wax. The paraffin block must then be microtome-sectioned, generating a paraffin ribbon, which is then mounted on a microscope slide. These steps serve to adhere the fixed cells to the surface of the glass microscope slide. The microtome sectioning and mounting steps are labor-intensive; there is no automation for these steps. The use of the tissue matrix eliminates the need for all of these steps.

The liquid matrix addresses this labor-intensive aspect in attaching non-adherent cell lines to microscope slides in a format that can withstand antigen retrieval and immersion in organic solvents, such as for immunohistochemical staining. Instead of all the aforementioned steps, cells can be formalin-fixed and then dispersed in the liquid matrix. None of the subsequent steps are then needed. The liquid matrix (containing cells) is dispensed onto a microscope slide or other solid surface. After the liquid matrix dries and solidifies, the cells are entrapped and adhered to the slide. Since the solidified matrix is porous to biological macromolecules, solvents, dyes, and other similarly sized compounds, the cells can be assayed/stained. If the cells are previously documented to express a particular protein, nucleic acid, or other macromolecule of relevance, then the cells can serve as a positive assay control.

The liquid matrix can also be used as an embedding medium for cells that have nothing to do with quality control. For example, it can be used as an embedding medium for cells derived from aspirates, blood samples, or biopsies. This more general use of the liquid matrix, for any small particulate matter, could pertain to any object worthy of staining or assaying. Moreover, it can be used for attachment to many solid surfaces beyond microscope slides. The exact shape or size of the solid surface is only limited by its suitability in the assay itself. For example, the liquid matrix can be coupled with a method for recovering rare circulating tumor cells from blood. After enrichment from blood, adhering the few cells to a glass slide is challenging. Such adherence is needed if the cells are to be analyzed by some type of stain such as an immunohistochemical stain.

The liquid matrix can also be used to help tissue sections adhere to microscope slides. Sometimes, tissue sections detach from the microscope slide during the various processing steps. Poorly fixed or fatty tissues are well known to be difficult in this regard. A simple process has been devised for improving tissue section adherence using the liquid matrix. After deparaffinization and re-hydration, 1-2 drops of liquid matrix are deposited on top of the tissue section (mounted on a glass slide). Then, the tissue section (with overlying liquid matrix) is covered with a small membrane that is cut to be slightly larger than the tissue section. By "membrane" is meant the types of materials that are often used for blotting, such as Southern or Western blotting. The membranes are typically made of PVDF, nylon, or nitrocellulose. Covering the tissue section with the membrane helped spread out the liquid matrix into a thin layer. At this point, there is a kind of "sandwich" comprised of the microscope slide on the bottom, membrane on the top, and the tissue section immersed in the liquid matrix in the middle. The slide is left to dry at room temperature for at least 5 minutes, after which the normal sequence of steps for immunostaining can continue. The first step is typically antigen retrieval, a high temperature treatment whereby the slides are immersed in a buffer. The membrane spontaneously fell off during this step, leaving the hardened liquid matrix covering the tissue section. Since the liquid matrix is porous to the immunostaining reagents, the tissue sections stain normally. The liquid matrix results in markedly improved adherence of the tissue section to the microscope slide.

Summarizing, the fact that the liquid matrix is dispensed as a liquid and hardens into a porous matrix suitable for many types of stains/assays renders it useful for holding any small particulate object in place while it is being analyzed in an assay. An advantage to using the liquid matrix is that it causes adhesion of beads or other small particulate matter to a microscope slide (or other surface) without the need for embedding the beads in a paraffin tissue block.

<u>Liquid matrix composition</u> The liquid matrix solution optimally comprises five distinct components: a carbohydrate, protein, solubilized collagen, a non-ionic detergent, and an anti-microbial preservative. For all of these components, there is a range of suitable concentrations. An exemplary concentration is provided for each.

The precise pH and buffer molar concentration is not critical. As an example, the following components can be added to a 50 mM sodium phosphate - citrate buffer, pH 6.0. (1) A carbohydrate such as 0.3% glycogen [Type II, from Oyster; Sigma-Aldrich, St. Louis, MO]. Suitable alternatives are 0.3% alpha-methyl cellulose (Amresco, Solon, OH) or 0.3% trehalose (Sigma-Aldrich, St. Louis, MO). Although alginates do not independently have the required properties (e.g., ability to withstand the heat of antigen retrieval or resistance to organic solvents), they can suffice (albeit less optimally) as the carbohydrate. The same is true for chitosan as well. (2) A protein, although not all promote adherence of beads to the glass slides. Suitable proteins include 0.3% keyhole limpet hemocyanin (KLH, Calbiochem/EMD Millipore, Billerica, MA), glutathione-S-transferase, and 0.3% goat gamma globulin (Sigma-Aldrich, St. Louis, MO). (3) Collagen.

Examples of collagen include solubilized type I (Rat tail collagen solution, GIBCO/Life Technologies, Grand Island, NY] or solubilized type IV collagen (Fluka/Sigma-Aldrich, St. Louis, MO), both of which would be added to a final concentration of approximately 0.01 - 0.2 mg/mL. (4) A non-ionic detergent. The non-ionic detergent helps promote spreading of the drop after being dispensed onto a microscope slide. An example is 0.0125% (final concentration) n-Octyl Beta-D-GlucoPyranoside (Sigma-Aldrich, St. Louis, MO). (5) An anti-microbial preservative, such as 0.1% sodium azide or 0.1% ProClin 300. These components can be dissolved in other buffers as well, such as phosphate buffered saline, pH 7.4.

Assay for Measuring Bead Retention To assess the role and optimal concentration for these liquid matrix components, a bead retention assay was used. Microbeads were suspended in the liquid matrix. Approximately one microliter of this suspension was then dispensed onto a microscope slide, in duplicate. The liquid matrix was then allowed to dry. Warming the slides, such as at 37 - 60 degrees Centigrade for at least one minute helped dry the liquid matrix, causing the liquid matrix to solidify. The slides were then subjected to the appropriate treatments as required for tissue deparaffinization, hydration, and antigen retrieval. These treatments are normally required if the slide also bears a tissue sample for staining. Deparaffinization includes immersion in xylene, decreasing grades of alcohol, and then water. The slides were then subjected to antigen retrieval, which may include incubation in a pressure cooker (often at temperatures at or approaching 120 degrees C) for 30 - 40 minutes. Lower temperatures (e.g., 90 - 100 C), without a pressure cooker, are also sometimes used for antigen retrieval. Various buffers have been described for antigen retrieval, but a common one is 0.1 M citrate buffer, pH 6.0. The slide on which the beads were mounted was then subjected to an immunohistochemical stain, involving the sequential application and removal of a series of reagents. At the end, another microliter of the same bead suspension in liquid matrix was placed on a slide (in duplicate) and dried, without being subjected to the steps of deparaffinization, antigen retrieval, and immunohistochemistry staining. The slides were then examined for the retention of beads in the liquid matrix. The number of beads per high power field (HPF, 40x objective, $10\times$ ocular) was then calculated as the mean + SD from six fields. The measurement was performed in duplicate.

In a representative experiment, the duplicate drops of beads that underwent deparaffinization, antigen retrieval, and staining, had 11.7 + 0.8 and $10.5 \pm 0.5$ beads per HPF (average 11.1). The duplicate drops of beads that did not undergo deparaffinization and antigen retrieval had $11.4 \pm 1.1$ and 12.2 + 1.3 beads per HPF (average 11.65). Therefore, the recovery of beads after deparaffinization and antigen retrieval was approximately 95%. An advantage of this system is that even less efficacious adhesives, with lower bead retention, can still provide the same important quality control feedback to the histotechnologist or pathologist examining the slide. For example, if the retention is only 50%, a manufacturer of this quality control device can simply add twice as many beads to the liquid matrix. The customer will still have an adequate number of adherent beads still attached to the microscope slide for microscopic evaluation. Generally, an adhesive with a low retention rate, such as less than 25% average retention, will be too erratic and unreliable in its bead retention to be clinically useful.

Induction of cross-links in the liquid matrix One deparaffinization - antigen retrieval solution to which the solidified matrix does not consistently withstand has been encountered. Few beads remain adherent after treatment. It is a buffer used in the Dako PT Link module, which is for deparaffinization and antigen retrieval. The buffer is proprietary; its composition is unknown. This problem was solved by incubating the solidified matrix (with its adherent beads) in a protein cross-linking solution such as formaldehyde or formalin. A ten minute incubation with formalin at 37 degrees C, or room temperature incubation for 30 minutes, both cause additional hardening of the liquid matrix so that the beads adhere even after deparaffinization and antigen retrieval with PT Link buffer. Treatment with formalin (or stock formaldehyde) does not interfere with subsequent immunostaining, indicating that the porosity of the solidified matrix is not materially affected by the treatment. Namely, even after formalin treatment, the matrix was still compatible with immunohistochemical assays. These findings indicate that cross-linking one or several components contained in the matrix helps strengthen the matrix. Other cross-linking agents, such as glutaraldehyde, would also be expected to have this effect.

Bead Stain Intensity Quantification

The beads can serve as a positive control for staining, as they bear the quality control moiety that produces a positive reaction in an assay. Their similarity to cells (in size and shape) makes the analysis of the beads to be similar to that of stained cells. Both the beads and sample for analysis are measured with optical magnification, such as by using a microscope. Beads with an irrelevant quality control moiety, or no quality control moiety, serve as a negative control. By comparing to the bead stain intensity of the same laboratory on previous days, or by comparing to other laboratories running the same assay, it is possible to determine if the assay is performing correctly. The stain intensity of the controls can be assessed visually, by eye, or with the aid of a computer image analysis program after image capture with a camera attached to the microscope. For example, a common method is to estimate stain intensity of a 0 - 3 scale (0: negative; 1: weak; 2: moderate; 3: strong).

Standardization of image quantification using color standard beads A source of variability in any assay is the imprecision of measurement. For assays performed using optical magnification, such as microscopy, the sources of color intensity measurement imprecision include the optical (microscopy) parameters and the method for measuring color intensity. Examples of optical parameters include the quality of the lenses and microscope settings (e.g., Kohler illumination). Examples of variables in measuring color intensity include the camera and its settings (e.g., exposure time). In order to increase measurement precision (the reproducibility of measurement), it is desirable to find methods to minimize the variability of these parameters from day to day and between labs. Doing so will foster intra- and inter-laboratory reproducibility of color intensity measurement.

A helpful method for standardizing color intensity measurement of microscope images is to include color standard beads. The bead preparation would thereby include both the beads bearing a quality control moiety (for staining in the assay) and the color standard beads. The former beads, bearing the quality control moiety, will become colored only after staining. For convenience, these beads are denoted as "test" beads. In contrast, the color standard beads are colored even without staining. Preferably, the color standard beads are of a similar color as the chromogen (or fluorochrome, chemiluminescent marker, or other optical parameter) that is used in the assay. For example, most immunohistochemical stains use diaminobenzidine (DAB) as the chromogen, which usually deposits as a brown pigment in stained samples. For immunohistochemical stains using DAB, a similar brown color standard bead was used. It is also desirable to distinguish the color standard beads from the test beads lest the observer misinterpret them as representing a positive reaction in the assay. For example, the color standard beads can be of a different physical dimension, such as different size or shape. For example, if the beads bearing quality control moieties are 7 - 8 microns in diameter, the color standard beads may be 3 - 4 microns in diameter. This difference facilitates their identification as distinct from the test beads. An example of beads that fulfill these criteria are sold under the commercial name "Dynabeads®" (Life Technologies, Grand Island, NY). A specific example includes Dynabeads® Pan Mouse IgG, catalog number 11041. The specific ligand that is attached is not important since the relevant optical characteristics are associated with the bead itself rather than the attached ligand.

The inclusion of color standard beads with the test beads helped compensate for the variability in color intensity measurement. Since the color standard beads have a pre-established and consistent color intensity, the color standard beads served as an internal reference point against which other color intensities (of test beads) were measured. This is true because the color standard beads' color intensity is dependent on the same optical and measurement variables as the test beads. If the color intensity measurement variables caused the stained beads to appear lighter or darker, then the color standard beads were similarly affected. Since the test beads and color standard beads were in the very same image, then normalizing the measured color intensity of the stained test beads against the measured color intensity of the color standard beads helped cancel out the optical and measurement variables. The color standard beads provided a fixed positive reference point of color intensity approximately corresponding to that of stained beads.

The unstained test beads (bearing a different, antigenically irrelevant quality control moiety) provided a fixed negative reference point of color intensity. These unstained beads are of the same composition and size as the stained test beads. Their color intensity, faint as it is, represents a color intensity baseline. With shorter camera exposure times, these unstained beads can appear darker. A longer camera exposure time causes their faint edges to become even fainter. With a sufficiently long camera exposure, the unstained test bead images can even blend in with the white background. To help control for this variability in measurement, it can be advantageous to use the unstained test beads' color intensity as a second internal color intensity reference standard against which to measure the stained test beads color intensity.

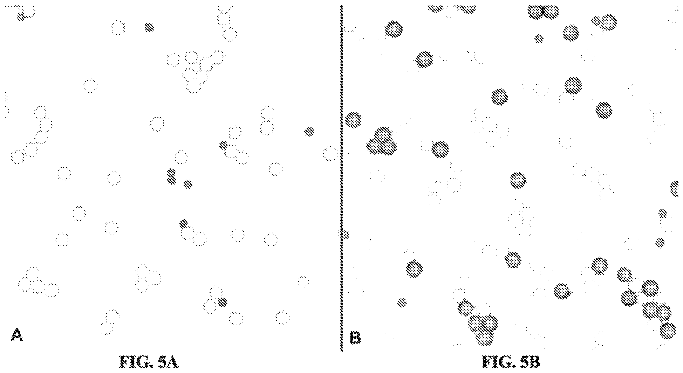

An example of these various beads is shown in FIG. 5A and 5B. FIG. 5A shows unstained test beads and color standard beads. In FIG. 5A, the only colored beads are the color standard beads, causing them to stand out against the background. In FIG. 5B, a portion of the test beads were stained because they bear the relevant quality control moiety for the stain that was applied. The stain intensity of the stained test beads was approximately the same as the color standard beads.

When using internal image color intensity reference standards, color intensity of the stained test beads can conveniently be expressed as a ratio. Typically, pixel color intensity in a camera with an 8 bit image depth is expressed on an absolute scale, from 0 - 255. White is usually at 255 and black at 0. Changing the various optical or camera settings can change the channel number, which is associated with an object appearing lighter or darker. By comparing the channel number of a desired set of pixels (corresponding to the test beads) to the channel number of the pixels containing one or both internal color intensity standards, the measurement becomes standardized. Approximately the same result will be measured regardless of the microscope or camera. A suitable equation for expressing color intensity according to this plan is:

$$\text{Test Bead Color Intensity} = \frac{\text{Unstained bead channel number} - \text{Stained test bead channel number}}{\text{Unstained bead channel number} - \text{Color standard bead channel number}}$$

In the numerator and denominator, the test bead or color standard bead are subtracted from the unstained bead channel number so as to express the difference as a positive integer. With this format, the test bead color intensity will be 1.0 if the pixel intensity of the test beads and color standard beads are identical. Higher numbers (>1.0) will indicate that the test beads are darker. With lower numbers, the color standard beads are darker.

An alternative method for calculating the test bead color intensity ignores the unstained beads. Such a calculation would actually be required if the image is acquired with such a long time exposure that the unstained beads are no longer visible. By contrast, the former equation (incorporating the unstained test bead channel number) will likely be a more accurate measure of the stained test bead color intensity under conditions whereby the unstained beads are more prominent, such as low camera exposure times. This alternative calculation is expressed as a simple ratio:

$$\text{Test Bead Color Intensity} = \frac{\text{Stained test bead channel number}}{\text{Color standard bead channel number}}$$

### Visual estimation of test bead color intensity

A visual (manual) method of quantifying test bead color intensity was developed that can be used without a camera or image analysis software. This system can be helpful in evaluating immunohistochemistry controls, whether the stain is performing as expected. It can also be helpful in comparing a particular laboratory's staining results against its peers, to verify that the stain is performed similarly so as to produce a comparable result from laboratory to laboratory. For convenience, 4 categories are created, designated 0 - 3+. The scoring criteria and examples of each are provided.

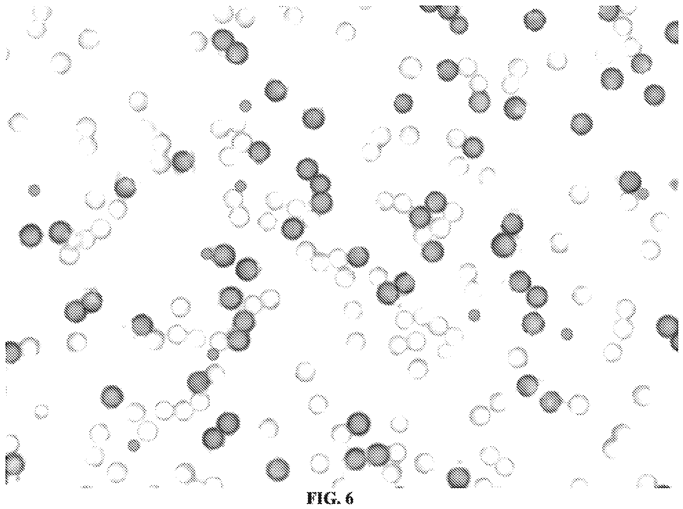

FIG. 6 illustrates an example of a 3+ (strong) immunostain on the cocktail of test beads (containing beads bearing both relevant an unrelated quality control moieties). The stain intensity of the rim is evaluated separately from the bead center. Light traveling tangential to the rim is subjected to a greater degree of bead surface area relative to the bead center. Therefore, the rims appear darker than the bead centers. In FIG. 6, the majority of beads have accentuated rim staining. This can best be appreciated by comparing the rim staining (of the stained test beads) to the rims of unstained test beads (bearing an antigenically irrelevant quality control moiety for the stain that was applied). In addition, the majority of bead centers are stained. Comparing the stained test beads to the color standard beads provides a simple gauge to assess stain intensity. The stained test beads are generally of equal or greater color intensity to the smaller color standard beads.

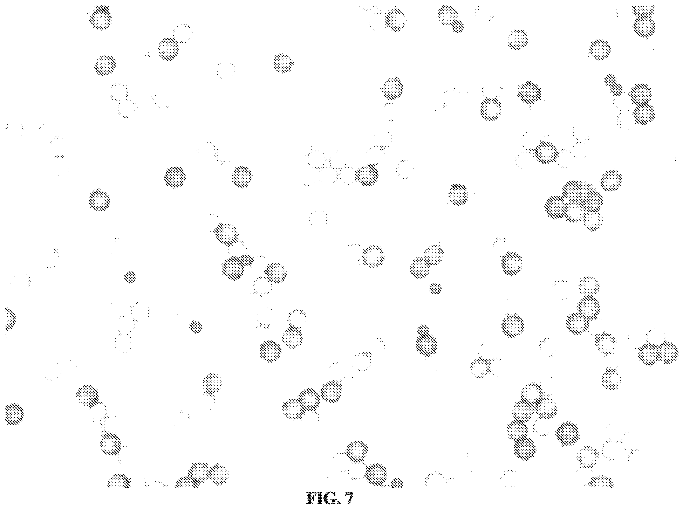

FIG. 7 illustrates an example of a 2+ (mild to moderate) immunostain on the cocktail of test beads. Bead rims are stained brown, but less intensely. Rim staining can be appreciated by comparing to unstained beads. The centers of many beads are tinted brown, but often not uniformly throughout the bead interior. Comparing the stained test beads to the color standard beads shows that the stained test beads are of a mildly-moderately weaker intensity than the small color control beads.

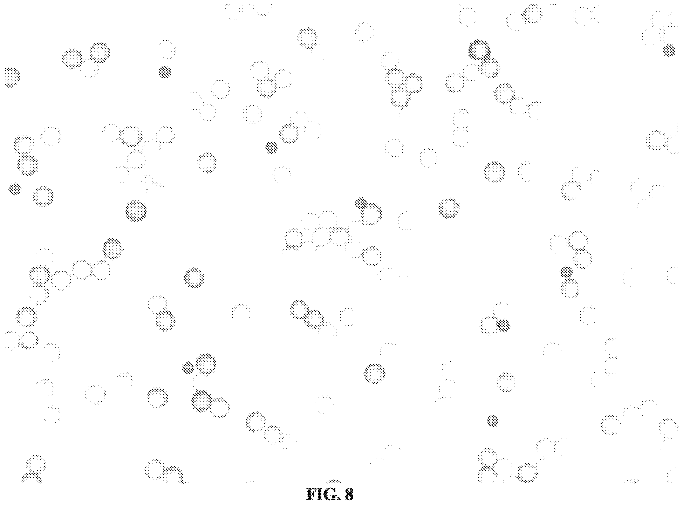

FIG. 8 illustrates an example of a 1+ (weak) immunostain on the cocktail of test beads. Bead rim staining distinguishes stained test beads from unstained test beads, but the bead rim color intensity is otherwise barely detectable. The beads' central interior appears washed out, without appreciable staining. The stained test bead color intensity is far below that of the color standard beads.

A score of 0 is used for no staining of the bead cocktail. In this situation, it would not be possible to distinguish stained test beads (bearing the relevant quality control moiety) from the unstained test beads.

In summary, the test beads can be quantified either with the benefit of instrumentation, such as a camera and image analysis software, or visually (without the aid of instrumentation). Both methods will work. The use of image quantification algorithms has yielded greater precision of measurement than visual estimation.

### Advantageous Features

<u>Convenience</u> Small bottles of beads, dispersed in liquid matrix, can be kept in a convenient location in the laboratory. Applying the control involves dispensing a small drop of beads (in liquid matrix) onto a desired slide and allowing it to dry. Thus, using the positive control is convenient and involves minimal additional labor. Importantly, the quality control does not require locating pathologic discard tissue blocks from previous patients and microtome sectioning, both of which are labor-intensive processes.

<u>Adaptable to manufacturing scale-up for national distribution</u> Beads can be prepared in large batches, for national or international distribution, resulting in every laboratory receiving approximately equivalent aliquots of beads. In this way, the quality control device is standardized across clinical laboratories. Different laboratories can compare their beads' color intensity and thereby draw valid comparisons about the efficacy of their staining relative to one another. One convenient method for comparing the staining of beads amongst laboratories is for participating laboratories to submit data, either in a quantitative format or images, to a central site. The data can then be analyzed, stratified by the particular assay, reagents and instrument used, and antigen retrieval protocol, so as to generate a mean and standard deviation for participating laboratories. Performance outside of a specified range, such as > $\pm3$ SD from the mean, is often considered as a threshold for unacceptable laboratory performance.

Flexibility of placement location on the slide Advantageously, the customer can place the control anywhere on the slide, such as alongside the patient sample. Most commonly, the technologist would first mount the tissue section or cell smear on the slide. Then, the technologist would consider which control ought to be placed on that particular microscope slide. The technologist then retrieves the appropriate bottle of beads (in liquid matrix) and dispenses a drop wherever desired on the same slide. Alternatively, the drop of bead control can be placed on a separate slide, without patient samples, such as for a batch control. If the beads are placed adjacent to a patient sample, then the beads serve as an on-slide control. The beads resemble cells in their approximate size and shape and are stained by the exact same set of reagents as the patient sample.

Advantageously, the customer can use whatever type of microscope slide is typically used. The types of microscope slides used for routine histology will sufffice.

Low cost of manufacture Advantageously, the cost of manufacture is diminished because the controls are not mounted or printed on slides by the manufacturer. Moreover, preparing the beads is inexpensive as a single test tube or bottle of beads (dispersed in liquid matrix) is convenient to handle, involves minimal labor, and yet can provide enough volume for many tests. Methods for packaging a liquid suspension (such as beads in a liquid matrix) into small bottles are also well known and relatively inexpensive.

Exemplary Methods of Use

Methods of using the quality control devices described herein are also encompassed by the present invention. These methods generally provide for evaluating the efficacy of an assay by evaluating the resulting color intensity. By performing the assay (e.g., a stain) on the quality control device, the assay result provides data as to whether the assay is functioning normally.

Using the invention as a check on assay sensitivity. Included in the present invention are methods for determining or monitoring the sensitivity of an assay. The phrase "sensitivity of an assay" in this context refers to the lowest concentration of analyte that can be detected with the assay in a biological sample. This can be important in verifying proper assay performance. If an assay is not functioning properly, then its sensitivity might worsen. Consequently, it may be desirable to have two or more levels of controls, each with different concentrations of the same quality control moiety. By using more than one level of control, the assay may cause some beads to be intensely colored, others faintly so, and yet others not at all.

For example, to determine the sensitivity of an immunohistochemical assay, the endpoint of detection was determined as the beads bearing the lowest antigen concentration that still produces a "1+" intensity on a 0 - 3+ scale. Such semiquantitative scales are common in the field of histopathology. A "1+" assessment (as determined by visual assessment) indicates a detectable signal, but at the lowest intensity level. By verifying that beads with a low concentration of quality control moiety produced a "1+" signal, the sensitivity of the assay is verified. If the stain intensity is too low to produce a detectable "1+" signal, then that indicates the existence of a staining problem to the laboratory technologist or pathologist. Image analysis software programs can be helpful in standardizing intensity measurements between individuals and from day to day. By performing this quality control assessment, the initial stages of assay failure can be identified. Although a low positive control ("1+" intensity) is commonly used as a sensitivity endpoint control, other control levels can be used for the purpose of assessing an assay's sensitivity. For example, if control beads that previously scored a "3+" now only score as barely detectable ("1+"), then that similarly provides a measure of worsening assay performance.

Such a sensitivity check can be performed on one slide per batch of slides. This confirms that the reagents are functional on that particular day. Moreover, it confirms that the assay was performed appropriately on the particular slide that was tested. Alternatively, a sensitivity check can be placed on every slide that contains a patient or biological sample. Placing a control on every slide verifies that the assay sensitivity is appropriate on every patient sample. A check on every slide verifies both reagent function and assay performance.

Using the invention as a calibrator. The present invention can also be used as a calibrator, in measuring the concentration of an analyte in a biological sample. To do so, the technologist performing the assay will simultaneously process the biological sample and a quality control device. When used as a calibrator, the device can comprise two or more groups of beads, each bearing different concentrations of a quality control moiety. If the concentrations of the quality control moieties on the calibrator beads are known, then measuring the resulting color intensity after processing the assay can facilitate the creation of a calibration curve. Typically, the concentration would be represented on the x axis of a graph and the color intensity (after staining) on the y axis. A calibration line represents the relationship between the intensity of the quality control moiety and the resulting color intensity. By interpolating the color intensity of the relevant cells in the sample to the calibration curve, the analyte concentration in the sample can be determined.

An alternative method of using the present invention as a calibrator is to use only one set of calibrator beads. Although the creation of a calibration line typically requires at least two points for a line, a single point calibration can be useful if there is a single, defined cutoff (threshold) that is clinically important. If the quality control moiety concentration on the beads is at or near the cutoff concentration (i.e., a staining intensity corresponding to a clinical decision threshold), then a single point calibration can be used to reliably identify the threshold for the purpose of evaluating patient samples. A single point calibration can also be practical if there are validation data indicating that the calibration line has a known consistent slope. The single point calibration point then serves to set the y intercept by establishing a single point on the line at a known quality control moiety concentration. A single point calibration can also work well if the calibration line passess through the origin (x-0, y=0).

Method for determining concentration of quality control moiety per bead To facilitate these various uses of the invention, it is desirable to know the concentration of the quality control moiety on the beads. Concentration data are helpful from a manufacturing standpoint, to foster manufacturing reproducibility. Per bead concentration data are also valuable to clinical laboratories, in determining sensitivity endpoints or calibration curves, as described previously.

A method for determining the concentration of the quality control moiety per bead is to attach a fluorochrome to it. A fluorochrome, such as fluorescein, can be attached to a peptide without interfering with antibody binding to the peptide. To do so, it is helpful to place the fluorochrome distant from the epitope, preferably spaced by at least 3 amino acids. This will help avoid the possibility of the fluorochrome interfering with the binding of antibody to the peptide epitope. The fact that the peptides have a single internal fluorochrome, and that fluorochrome has an established spectrophotometric absorptivity (also known as "extinction coefficient"), simplifies the calculation. The calculation involves two steps: (1) measuring the molar quantity of fluorochrome attached to an aliquot of beads and (2) enumerating the number of beads in the aliquot. From these two measures, the fluorochrome concentration per bead can be calculated. Since there is a single fluorochrome per peptide, the fluorochrome concentration per bead is also the peptide concentration per bead.

In order to measure the molar quantity of a fluorochrome using the spectrophotometric extinction coefficient, the peptide must first be released from the bead in a soluble form. The peptide (containing a fluorochrome) can be released if it was initially cross-linked to the bead with a cleavable cross-linker, such as DTSSP (3,3'-Dithiobis[sulfosuccinimidylpropionate]). DTSSP is a commercially available (Pierce Chemical Co., part of ThermoFisher Corp., Rockford, IL) water-soluble homobifunctional cross-linker for amine groups (aminosilane on the bead to a terminal or epsilon amine on the peptide). DTSSP has an internal disulfide link. Covalent coupling of fluorochrome-conjugated peptide (or other quality control moiety) is performed using DTSSP as per the manufacturer's instructions. Treatment with 50mM dithiothreitol (DTT) cleaves the disulfide link and releases the peptide from the bead. To cleave the DTSSP cross-link, incubate the beads in 20-50mM DTT at 37°C for 30 minutes. Percent release of peptide from the beads can be calculated using a flow cytometer or fluorescence microscope with an attached camera and image analysis capability, measuring bead fluorescence before and after DTT cleavage. Baseline fluorescence of the beads without any cross-linked moieties is subtracted from both bead measurements. The free peptide solution, collected after treatment with DTT, is then analyzed in a spectrophotometer. If fluorescein is the fluorochrome, then the absorbance is measured at 490 nm wavelength. DTT solution represents the spectrophotometer blank solution. It is important to verify that the quality control moiety without the fluorochrome has no appreciable absorption at the wavelength; all or most of the light absorption is therefore due to the fluorochrome. If the quality control moiety absorbs appreciable amounts of light without the fluorochrome, then the molar extinction coefficient for the fluorochrome may not apply. Using the absorbance reading, it is then straightforward to calculate the molar concentration of fluorescein, based on the Beer-Lambert equation:

$$\text{Absorbance} = \text{molar absorptivity x path length x concentration}$$

The molar absorptivity (extinction coefficient) of fluorescein at 490 nm in a 200 mM phosphate buffered solution at pH 7.4 is 76,000 $M^{-1}$ $cm^{-1}$ (see, for example, MC Mota, P Carvalho, J Ramalho & E Leite. Spectrophotometric analysis of sodium fluorescein aqueous solutions. Determination of molar absorption coefficient. International Ophthalmology 1991; 15:321-326. If using a spectrophotometer with a 1 cm wide cuvette, then the concentration of the peptide (bearing a single fluorescein) is:

$$\text{Concentration (moles/liter)} = \underline{\text{Molar absorptivity}}$$
$$\text{Molar extinction coefficient}$$

The number of moles of peptide can then be calculated by multiplying the volume of the solution by the concentration. This resulting number represents the total number of moles of peptide that were attached to the beads. Dividing that quantity by the number of beads in the aliquot yields the average molar concentration per bead.

These beads (using the cleavable cross-linker) can then serve as a set of peptide calibrator beads. It is helpful to generate a series of peptide calibrator beads with varying concentrations of the relevant peptide per bead. To generate beads with decreasing concentrations of covalently bound relevant peptide, use varying concentrations of peptide during the cross-linking step to the beads. It can be helpful to mix the relevant peptide with an irrelevant one so that the total peptide concentration remains constant. For example, if the relevant peptide comprises only 50% of the total peptide concentration, then a 50% decrement in staining intensity is expected. Including another (antigenically irrelevant) peptide in the conjugation mix to beads provides for a more predictable decrement in peptide concentration (as compared to diluting the peptide without an irrelevant peptide). The irrelevant peptide should not bear the same fluorochrome that is being used for calculating concentration. The fluorescence intensity per bead (after the conjugation reaction with peptide is complete) can then be directly correlated to the concentration of the antigenically relevant peptide. Although peptides have been described as the preferred quality control moiety for immunohistochemistry, these principles apply to any quality control moiety.

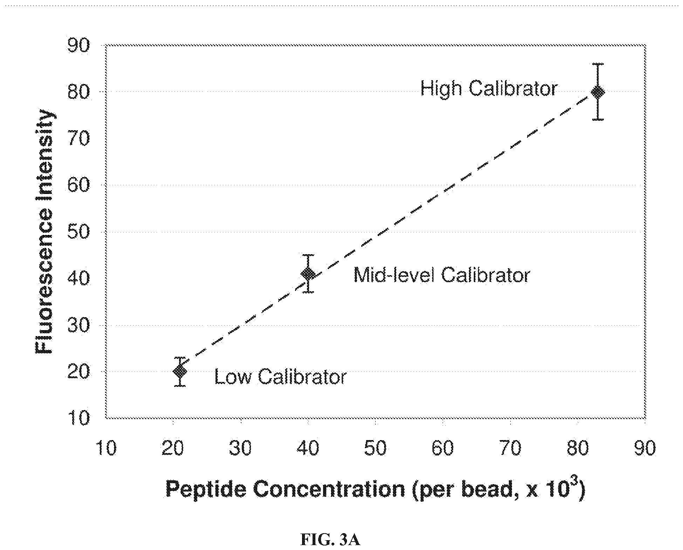

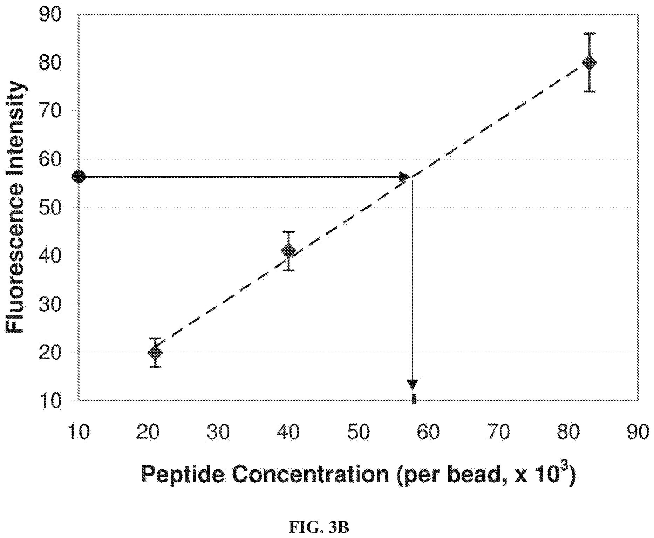

For a manufacturer of this device, it may be helpful to establish a calibration curve for a master lot of beads, identifying the relationship between fluorescence per bead and the quality control moiety per bead molar concentration. The calibration curve can be established by plotting the measured fluorescence and calculated concentration on a graph (FIG. 3A). The concentration is plotted on the x axis and fluorescence on the y axis. With this calibration curve, the concentration per bead from newly manufactured lots of beads can be easily determined. First, the bead fluorescence is measured, either using a flow cytometer or fluorescence microscope. Then, the point along the y axis corresponding to the measurement is identified. FIG. 3B provides an example, with the solid dot along the y axis representing the measured fluorescence. The corresponding x axis value is then determined by tracing along the graph so as to identify the x axis value below the point of intersection with the regression line. An equivalent method would be to calculate the regression line, in the form $y = mx+b$, whereby m is the slope and b is the y intercept of the line. Then, by inserting the fluorescence value as "y" in the equation, solving for "x" will identify the corresponding per bead concentration.

The peptide-conjugated beads can be divided into aliquots and frozen (-80 degrees C) for long-term storage. From one of the aliquots, one can measure the concentration of fluorochrome after releasing the peptide with DTT, as already described. These calibrator beads are intended for comparison to any other peptide bead product, to estimate peptide concentration per bead. With a known calibrator set of peptide beads, peptide concentrations on manufacturing production lots can be readily calculated. Deriving the peptide concentration per bead on a routine manufacturing lot involves two steps. First, one measures the fluorescence intensity of the calibrator beads, such as by using flow cytometry or fluorescence microscopy with image analysis. It is helpful to graph the peptide concentration per bead on the x axis and fluorescence intensity (such as measured by flow cytometry) on the y axis. A linear regression line can be calculated from the data points. The line resulting from this graph represents a calibration curve. Then, as a second step, the fluorescence intensity of the new beads is measured in the new production lot (that are of an unknown per bead peptide concentration). This fluorescence intensity represents the y value in the calculated calibration regression line. By inserting the fluorescence intensity value into the equation for the regression line of the calibration curve, the mean peptide concentration per bead can be calculated. This capability may help promote the development of reproducible calibration standards in the field of IHC. This system of calculations is adaptable to any type of quality control moiety, not just peptides, provided that there is a known number of fluorochromes per quality control moiety. For example, the same could be accomplished with DNA or proteins (other than synthetic peptides).

Use as a Quality Control. A method for verifying the proper performance of an assay performed on a microscope slide can include simultaneously processing the biological sample and this herein described quality control device in the assay, wherein processing results in a detectable signal (e.g., a colorimetric signal) produced by the analyte and by the quality control moiety. The fact that the quality control moiety caused a colorimetric signal to ensue, at an expected level of intensity, verified that the assay was correctly executed. This is especially important in instances where the biological sample yields a negative result, i.e., no color development. The fact that the quality control moiety yielded a positive reaction established that the result on the sample was a true negative and not due to errors in the procedure or problems with reagent quality. This method includes the step of quantification of the color reaction on the quality control moiety. This quantification can be by use of a computer image analysis software algorithm, such as with MatLab. Alternatively, the quantification can be a semiquantitative visual estimate, as measured by an observer. By measuring and recording the day-to-day color intensity associated with the quality control result, an operator can track assay performance over time. Changes in color intensity may reflect alterations in the assay. Such graphs are often termed "Levey-Jennings" charts. The graphs are a tool for statistical analysis of laboratory assays. Changes beyond 2 or 3 SD from the mean can represent a flag to the technical staff, signifying the possible need for further evaluation of assay performance.

Formalin fixation of peptide-conjugated beads

In serving as an immunohistochemistry quality control device, it is desirable that peptide-coated beads are also able to evaluate the efficacy of antigen retrieval (AgR). AgR is a process that is considered to unmask/recover antibody epitopes that are otherwise lost after formalin fixation. Antigen retrieval is an essential step for virtually all immunohistochemical stains after formalin fixation. To provide a quality control device for evaluating the efficacy of antigen retrieval, the peptide-coated beads must first be masked in a similar fashion as occurs to tissue samples during formalin fixation.

Formalin fixation of tissues and cells were simulated by cross-linking other proteins to the peptide epitope, thereby resulting in steric interference of antibody binding to the peptide epitope. The technical challenge is in finding a way to formaldehyde cross-link proteins to peptides. If the peptide-coated beads are re-suspended in a solution containing protein and formaldehyde, the favored cross-linking reaction will be for the proteins to cross-link to each other. Compared to peptides, each soluble protein molecule is larger and has a more diverse set of amino acid side chains with which to cross-link. Moreover, whereas the peptides are immobilized on the bead surface, the soluble proteins are physically unrestricted and therefore better able to chemically react with one another.

One previous solution to this problem included using gaseous formaldehyde. A protein was deposited on top of a glass microscope slide that had a covalently attached peptide. By depositing the protein directly on top of the peptide (attached to a glass slide), the two were brought into close contact. If liquid formaldehyde was added, then it would have dissolved the protein, dislodging it away from the peptide and frustrating the goal of cross-linking the peptide and protein. To solve that problem, gaseous formaldehyde (vapor) was used. That solution is not practical in the context of small beads suspended in solution. Gaseous formaldehyde will not penetrate into solution.

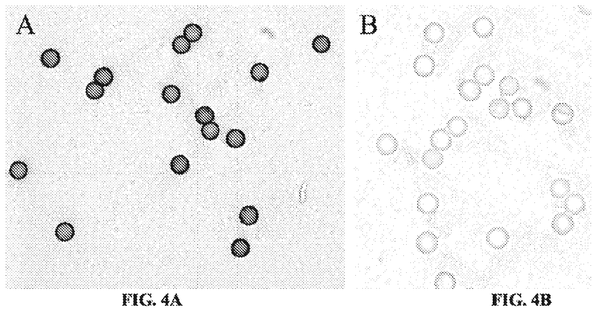

To solve the problem of cross-linking peptide and protein on the surface of a glass bead, a condition was created whereby the soluble protein molecules were forced into close proximity to the bead-immobilized peptides. Specifically, soluble protein and liquid formaldehyde were added to the beads (in a small volume, sufficient to wet the beads) and the mixture was incubated so as to cause the liquid to evaporate. The term "liquid formaldehyde" can be of varying concentrations of formaldehyde and should not be interpreted to imply only a stock 37% formaldehyde. As the volume of liquid decreases, the proteins in solution are deposited close or onto the bead surface, in proximity to the attached peptides. Simple methods for causing formaldehyde evaporation include incubation at temperatures above room temperature and leaving the container unsealed. This facilitates formaldehyde-induced protein cross-linking to the peptides (for example, on the beads), thereby resulting in diminished immunoreactivity and immunohistochemical staining intensity. A diminished stain intensity was evidenced in an immunohistochemical assay as a reduced color intensity. Antigen retrieval reversed the process, restoring immunoreactivity, resulting in a positive immunohistochemical stain. FIG. 4 shows representative examples of immunohistochemical staining on formaldehyde-fixed beads after antigen retrieval (FIG. 4A, left) and before antigen retrieval (FIG. 4B, right). These particular beads bear an epitope for a human estrogen receptor antibody epitope. FIG. 4B demonstrates a loss of immunoreactivity and negative reaction. FIG. 4A demonstrates restoration of immunoreactivity and a positive reaction after antigen retrieval.

An exemplary procedure to create formaldehyde-fixed beads is described. First, the peptide-coated beads are re-suspended in a protein containing solution. By incubating the peptide-coated beads with one or more proteins, the proteins weakly and non-covalently adhere to the beads. For example, an exemplary protocol is to re-suspend glass beads (covalently coated with peptide) in a solution of 10 mg/mL casein in 0.2M Potassium phosphate buffer pH 8.5 and incubate at room temperature for 30-60 minutes. It is possible that for this first step, soluble protein (casein) becomes at least weakly associated with the bead surface during this incubation. The beads are then pelleted by centrifugation (e.g., approximately 2000 x g for one minute) and almost all of the supernatant (containing protein) is removed. For example, from a 1 mL suspension of beads, a residual volume of 50 microliters of the protein solution can be left. Most of the protein is removed but a small residual remains weakly associated with the bead surface. The beads are left at 37 C for at least 4 hours in an unsealed container so that the protein solution evaporates, leaving dry beads. This step can be extended overnight. By evaporating the residual protein solution, the previously dissolved proteins become (non-covalently) deposited on the bead surface. To the dried beads is added a small amount of 30% formaldehyde in 0.2 M Potassium phosphate buffer (pH 8.5). The initial addition of liquid formaldehyde to the bead pellet may cause dissociation and solubilization of the proteins that were previously weakly adherent to the bead surface. However, as the fluid evaporates, the soluble protein is again deposited at the bead surface, in proximity to the attached peptides. For example, for a 1 mL bead pellet, approximately 50 microliters of formaldehyde solution is added, which is sufficient to wet the beads. The volume of formaldehyde (or formalin) to add is approximately 0.01 - 0.1 times the volume of the pelleted beads. The beads are then gently dislodged by tapping the test tube, allowing the formaldehyde to penetrate into the bead pellet. It is helpful to de-aggregate the bead pellet with tapping or gentle swirling of the tube. The tube is left uncapped in a 37 - 40 C degree oven. By warming the tube, the formaldehyde cross-linking reaction is accelerated and the liquid formaldehyde evaporates. The beads in formaldehyde are left at either 37 - 40° C for a minimum of 4 hours or overnight. The tube should be left uncapped so that the formaldehyde evaporates. In the presence of formaldehyde, the proximity of proteins and peptides on the bead surface favors covalent cross-linking of the two. Then, the beads are warmed at 57 - 60° C for 15 minutes. After the beads dry, 400 microliters of 0.2M $KH_2PO_4$ buffer is added to the beads and mixed well by vortex mixing. The beads are disaggregated by mixing, grinding, or sonicating the beads. The beads are then centrifuged for 2 minutes and the supernatant is removed with a 1 ml Eppendorf pipetter. The supernatant is discarded. The beads are re-suspended in 1 ml of the KH2PO4 (0.2M) buffer with 0.05% Casein.

After completing this reaction, the beads lost most or all of their immunoreactivity to the relevant antibody.

Immunohistochemical staining yielded a weak (low) or negative colorimetric reaction in the assay, an example of which is shown in FIG. 4B. This finding is consistent with the view that the formaldehyde cross-linking reaction masked the peptide epitopes. After antigen retrieval, which involves heating the beads (typically mounted on a microscope slide), the beads were again highly immunoreactive with the relevant antibody (FIG. 4A). The nature of the cross-links induced by formaldehyde are similar to those created by formalin in tissue sections; both are reversible after antigen retrieval. Therefore, formalin-fixed beads (coated with peptide epitopes) can serve as test substrates that also evaluate the process of antigen retrieval. If antigen retrieval is inadequate, the formaldehyde cross-linking reaction will not be adequately reversed and proteins will still sterically block antibody binding to the peptide epitope. Inadequate antigen retrieval will produce a weak (low) or negative assay result.

Other embodiments are within the scope of the following claims.

**Claims**

1.  A quality control or calibrator device comprising a quality control moiety with an attached fluorochrome wherein the quality control moiety is reactive in an assay performed on a microscope slide and capable of causing a positive test result and the concentration of the quality control moiety is determined by measuring the concentration of the fluorochrome; wherein the quality control moiety is attached to particulate matter and the particulate matter is adhered to the microscope slide.

2.  A control or calibrator device as described in claim 1 wherein said assay produces an optical result that is measured with the aid of a microscope by analyzing a color or fluorescent light in the spatial context of a cell.

3.  A control or calibrator device as described in claims 1 - 2 wherein said assay is an immunohistochemical stain.

4.  A control or calibrator device as described in claims 1-3 wherein the particulate matter are beads.

5.  A control or calibrator device as described in claim 4, comprising two or more groups of beads, each bearing different concentrations of a quality control moiety.

6.  A control or calibrator device as described in claims 1 - 5 wherein the fluorochrome is fluorescein.

7.  A control or calibrator device as described in claims 1 - 6 wherein the quality control moiety is a peptide, protein, or nucleic acid.

8.  A method of using the device as described in claims 2 - 7 comprising comparing the intensity of the optical result to an acceptable pre-established range and then determining if the assay is performing correctly.

9.  A method for using the device of claims 2 - 7 comprising creating a calibration curve representing the relationship between the concentration of the quality control moiety and the intensity of the optical result.

10. A method as described in claim 9 further comprising determining the concentration of an analyte in a sample with cells by processing the sample in said assay and interpolating the intensity of the assay optical result in the spatial context of the cellular analyte onto the calibration curve.

11. A method for using the device of claims 1 - 7 comprising two of more quality control moiety concentrations that are processed in the assay and identifying the lowest concentration that can be detected.

**Patentansprüche**

1.  Qualitätskontroll- oder Kalibratorvorrichtung, umfassend einen Qualitätskontrollteil mit einem angebundenen Fluorochrom, wobei der Qualitätskontrollteil in einem Assay reaktiv ist, der auf einem Objektträger durchgeführt wird und in der Lage ist, ein positives Testergebnis zu bewirken, und die Konzentration des Qualitätskontrollteils durch Messen der Konzentration des Fluorochroms bestimmt wird; wobei der Qualitätskontrollteil an Feststoffteilchen angebunden ist und die Feststoffteilchen an dem Objektträger haften.

2.  Qualitätskontroll- oder Kalibratorvorrichtung nach Anspruch 1, wobei der Assay ein optisches Ergebnis erzeugt, das mithilfe eines Mikroskops durch Analysieren einer Farbe oder eines Fluoreszenzlichts in dem räumlichen Kontext einer Zelle gemessen wird.

3. Qualitätskontroll- oder Kalibratorvorrichtung nach Anspruch 1-2, wobei der Assay eine immunhistochemische Färbung ist.

4. Qualitätskontroll- oder Kalibratorvorrichtung nach Anspruch 1-3, wobei die Feststoffteilchen Beads sind.

5. Qualitätskontroll- oder Kalibratorvorrichtung nach Anspruch 4, umfassend zwei oder mehrere Gruppen von Beads, wobei jede verschiedene Konzentrationen eines Qualitätskontrollteils trägt.

6. Qualitätskontroll- oder Kalibratorvorrichtung nach Anspruch 1-5, wobei das Fluorochrom Fluorescein ist.

7. Qualitätskontroll- oder Kalibratorvorrichtung nach Anspruch 1-6, wobei der Qualitätskontrollteil ein Peptid, Protein, oder eine Nukleinsäure ist.

8. Verfahren zur Verwendung der Vorrichtung nach Anspruch 2-7, umfassend Vergleichen der Intensität des optischen Ergebnisses mit einem akzeptablen zuvor bestimmten Bereich und dann Bestimmen, ob der Assay korrekt funktioniert.

9. Verfahren zur Verwendung der Vorrichtung nach Anspruch 2-7, umfassend Erstellen einer Kalibrierkurve, welche das Verhältnis zwischen der Konzentration des Qualitätskontrollteils und der Intensität des optischen Ergebnisses darstellt.

10. Verfahren nach Anspruch 9, ferner umfassend Bestimmen der Konzentration eines Analyten in einer Probe mit Zellen durch Bearbeiten der Probe in dem Assay und Interpolieren der Intensität des optischen Ergebnisses des Assays in dem räumlichen Kontext des zellulären Analyten auf die Kalibrierkurve.

11. Verfahren zur Verwendung der Vorrichtung nach Anspruch 1-7, umfassend zwei oder mehrere Qualitätskontrollteilkonzentrationen, die in dem Assay bearbeitet werden, und Identifizieren der geringsten Konzentration, die nachgewiesen werden kann.

## Revendications

1. Dispositif de contrôle de la qualité ou d'étalonnage comprenant une fraction de contrôle de la qualité avec un fluorochrome attaché, dans lequel la fraction de contrôle de la qualité est réactive dans un dosage effectué sur une lame de microscope et peut provoquer un résultat de test positif et la concentration de la fraction de contrôle de la qualité est déterminée par mesure de la concentration du fluorochrome ; dans lequel la fraction de contrôle de la qualité est attachée à de la matière particulaire et la matière particulaire est collée à la lame de microscope.

2. Dispositif de contrôle ou d'étalonnage selon la revendication 1 dans lequel ledit dosage produit un résultat optique qui est mesuré à l'aide d'un microscope par analyse d'une couleur ou de lumière fluorescente dans le contexte spatial d'une cellule.

3. Dispositif de contrôle ou d'étalonnage selon les revendications 1 et 2 dans lequel ledit dosage est une coloration immunohistochimique.

4. Dispositif de contrôle ou d'étalonnage selon les revendications 1 à 3 dans lequel la matière particulaire consiste en des billes.

5. Dispositif de contrôle ou d'étalonnage selon la revendication 4, comprenant au moins deux groupes de billes, chacun portant des concentrations différentes d'une fraction de contrôle de la qualité.

6. Dispositif de contrôle ou d'étalonnage selon les revendications 1 à 5 dans lequel le fluorochrome est la fluorescéine.

7. Dispositif de contrôle ou d'étalonnage selon les revendications 1 à 6 dans lequel la fraction de contrôle de la qualité est un peptide, une protéine, ou un acide nucléique.

**8.** Procédé d'utilisation du dispositif tel que décrit dans les revendications 2 à 7 comprenant la comparaison de l'intensité du résultat optique à une plage acceptable préétablie puis la détermination que le dosage se déroule correctement ou non.

**9.** Procédé d'utilisation du dispositif des revendications 2 à 7 comprenant la création d'une courbe d'étalonnage représentant la relation entre la concentration de la fraction de contrôle de la qualité et l'intensité du résultat optique.

**10.** Procédé tel que décrit dans la revendication 9 comprenant en outre la détermination de la concentration d'un analyte dans un échantillon avec des cellules par traitement de l'échantillon dans ledit dosage et interpolation de l'intensité du résultat optique de dosage dans le contexte spatial de l'analyte cellulaire sur la courbe d'étalonnage.

**11.** Procédé d'utilisation du dispositif des revendications 1 à 7 comprenant au moins deux concentrations de fraction de contrôle de la qualité qui sont traitées dans le dosage et l'identification de la concentration la plus basse qui peut être détectée.

10 μm

**FIG. 1**

10 μm

FIG. 2

**FIG. 3A**

**FIG. 3B**

FIG. 4A

FIG. 4B

**FIG. 5A**

**FIG. 5B**

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61901394
- US 7011940 B

- US 6855490 B

**Non-patent literature cited in the description**

- **RHODES, A. et al.** Evaluation of HER2/neu immunohistochemical assay sensitivity and scoring on formalin-fixed and paraffin-processed cell lines and breast tumors. *Anat. Pathol.,* 2002, vol. 118, 408-417
- **RHODES, A. et al.** A formalin-fixed, paraffin-processed cell line standard for quality control of immunohistochemical assay of HER-2/neu expression in breast cancer. *Amer. J. Clin. Pathol.,* 2002, vol. 117, 81-89
- **RIERA, J. et al.** Use of cultured cells as a control for quantitative immunocytochemical analysis of estrogen receptor in breast cancer. *Amer. J. Clin. Pathol.,* 1999, vol. 111, 329-335
- **HAMMOND, M. et al.** Standard reference material for Her2 testing: Report of a National Institute of Standards and Technology-sponsored consensus workshop. *Appl. Immunohistochem. & Mol. Morphol.,* 2003, vol. 11 (2), 103-106
- **SOMPURAM, S ; V KODELA ; H RAMANATHAN ; C WESCOTT ; G RADCLIFFE ; SA BOGEN.** Synthetic peptides identified from phage-displayed combinatorial libraries as immunodiagnostic assay surrogate quality control targets. *Clin. Chem.,* 2002, vol. 48 (3), 410-420
- **SOMPURAM, S ; V KODELA ; K ZHANG ; H RAMANATHAN ; G RADCLIFFE ; P FALB ; SA BOGEN.** A novel quality control slide for quantitative immunohistochemistry testing. *J. Histochem. Cytochem.,* 2002, vol. 50, 1425-34

- **SOMPURAM, S ; V KODELA ; H RAMANATHAN ; C WESCOTT ; G RADCLIFFE ; SA BOGEN.** Synthetic peptides identified from phage-displayed combinatorial libraries as immunodiagnostic assay surrogate quality control targets. *Clinical Chemistry,* 2002, vol. 48 (3), 410-420
- **SOMPURAM SR ; K VANI ; LJ HAFER ; SA BOGEN.** Antibodies Immunoreactive With Formalin-Fixed Tissue Antigens Recognize Linear Protein Epitopes. *Am. J. Clin. Pathol,* 2006, vol. 125, 82-90
- **A-S SCHROHL ; H.C. PEDERSEN ; S.S. JENSEN.** Human epidermal growth factor receptor 2 (HER2) immunoreactivity: specificity of three pharmacodiagnostic antibodies. *Histopathology,* 2011, vol. 59, 975-983
- *Biochem Eng J,* 2013, vol. 79, 33-40
- **KING S.M. ; QUARTUCCIO S. ; HILLIARD T.S. ; INOUE K. ; BURDETTE J.E.** Alginate Hydrogels for Three-Dimensional Organ Culture of Ovaries and Oviducts. *JoVE,* 2011, vol. 52, 2, http://www.jove.com/details.php?id=2804
- **S-R SHI ; C LIU ; J PEREZ ; CR TAYLOR.** Protein-embedding Technique: A Potential Approach to Standardization of Immunohistochemistry for Formalin-fixed, Paraffin-embedded Tissue Sections. *J Histochem Cytochem,* 2005, vol. 53, 1168
- **MC MOTA ; P CARVALHO ; J RAMALHO & E LEITE.** Spectrophotometric analysis of sodium fluorescein aqueous solutions. Determination of molar absorption coefficient. *International Ophthalmology,* 1991, vol. 15, 321-326